# EUROPEAN PATENT SPECIFICATION

(11) **EP 0 944 603 B1**
(45) Date of publication and mention of the grant of the patent: **19.02.2003**
(21) Application number: 97949622.1
(22) Date of filing: 25.11.1997
(51) Int. Cl.: C07D 233/50, C07D 401/12, C07D 403/12, C07D 405/12, C07D 417/12, C07D 215/38

(54) **PROCESS FOR MAKING 2-AMINO-2-IMIDAZOLINE, GUANIDINE, AND 2-AMINO-3,4,5,6-TETRAHYDROPYRIMIDINE DERIVATIVES**
VERFAHREN ZUR HERSTELLUNG VON 2-AMINO-2-IMIDAZOLINEN, GUANIDINEN, UND 2-AMINO-3,4,5,6-TETRAHYDROPYRIMIDINEN
PROCEDE DE FABRICATION DE DERIVES 2-AMINO-2-IMIDAZOLINE, GUANIDINE, ET 2-AMINO-3,4,5,6-TETRAHYDROPYRIMIDINE

(30) Priority: 25.11.1996 US 34318 P
(43) Date of publication of application: 29.09.1999
(73) Proprietor: THE PROCTER & GAMBLE COMPANY, Cincinnati, Ohio 45202 (US)
(72) Inventor: GODLEWSKI, Michael, South Plymouth, NY 13844 (US); KLOPFENSTEIN, Sean, Rees, Loveland, OH 45140 (US); MUNDLA, Screenivasa, Reddy, Norwich, NY 13815 (US); SEIBEL, William, Lee, Hamilton, OH 45011 (US)
(74) Representative: Nargolwalla, Cyra
(86) International application number: US9721646
(87) International publication number: WO98023595

(56) References cited:
- EP-A- 0 012 822
- DE-A- 1 670 807
- BERGMANN J ET AL: "SYNTHESIS AND STRUCTURE-ACTIVITY RELATIONSHIP OF SOME NEW BETA-BLOCKING AGENTS WITH POSSIBLE ALPHA-ADRENORECEPTOR ACTIVITY" ARCHIV DER PHARMAZIE., vol. 323, no. 7, July 1990, WEINHEIM DE, pages 387-391, XP002059755
- CHAPLEO C B ET AL: "HETEROAROMATIC ANALOGUES OF THE ALPHA2-ADRENORECEPTOR PARTIAL AGONIST CLONIDINE" JOURNAL OF MEDICINAL CHEMISTRY, vol. 32, no. 7, July 1989, pages 1627-1630, XP000601554 cited in the application
- NAJER H ET AL : "UNE NOUVELLE SYNTHESE DES ARYLAMINO-2 IMIDAZOLIDINES" BULLETIN DE LA SOCIETE CHIMIQUE DE FRANCE., 1961, PARIS FR, pages 2114-2126, XP002059756

## Description

### FIELD OF THE INVENTION

The present invention relates to chemical processes for making compounds useful in the treatment of various medical disorders, including respiratory disorders, ocular disorders, gastrointestinal disorders, nasal decongestion, hypertension, migraine, disorders associated with sympathetic nervous system activity, and substance abuse. In particular, the processes of this invention are useful for making 2-amino-2-imidazoline derivatives, guanidine derivatives, and 2-amino-3,4,5,6-tetrahydropyrimidine derivatives.

### BACKGROUND OF THE INVENTION

The present invention relates to processes for making 2-amino-2-imidazoline derivatives, guanidine derivatives, and 2-amino-3,4,5,6-tetrahydropyrimidine derivatives (all herein collectively described as "2-amino-2-derivatives"). Such derivatives are useful for the treatment of many medical disorders including, for example, respiratory disorders, ocular disorders, gastrointestinal disorders, nasal decongestion, hypertension, migraine, disorders associated with sympathetic nervous system activity, and substance abuse. One of the most widely known of these derivatives is clonidine, an alpha-2-adrenoreceptor agonist and antihypertensive agent. Iopidine is also a known alpha-2-adrenoreceptor agonist useful in reducing intra-ocular pressure: 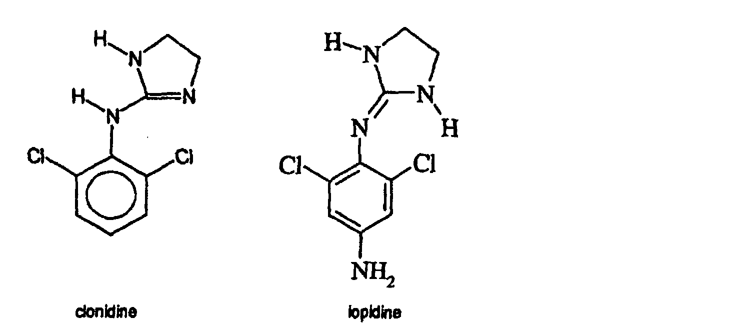 Clonidine, disclosed in U.S. Patent No. 3,202,660 (1965) to Boehringer, Ing.; Iopidine, disclosed in U.S. Patent No. 4,517,199 (1985) to Alcon; Timmermans, P.B.M.W.M., de Jonge, A., Thoolen, M.J.M.C., Wilffert, B., Batink, H., van Zwieten, P.A., "Quantitative Relationships between α-Adrenergic Activity and Binding Affinity of α-Adrenoceptor Agonists and Antagonists", Journal of Medicinal Chemistry, Vol. 27 (1984) pp. 495-503; Physician's Desk Reference (50th ed., 1996).

Therapeutic indications of alpha-2-adrenoreceptor agonists have been discussed in the literature. Ruffolo, R.R., Nichols, A.J., Stadel, J.M., and Hieble, J.P.; "Pharmacologic and Therapeutic Applications of Alpha-2-Adrenoceptor Subtypes", Annual Review of Pharmacology & Toxicology, Vol. 32 (1993) pp. 243-279.

Further information regarding alpha adrenergic receptors, agonists and antagonists, in general, are disclosed in the following references: Timmermans, P.B.M.W.M., Chiu, A.T., and Thoolen, M.J.M.C., "12.1 α-Adrenergic Receptors", Comprehensive Medicinal Chemistry, Vol. 3, Membranes & Receptors, P. G. Sammes & J. B. Taylor, eds., Pergamon Press (1990), pp. 133-185; Timmermans, P.B.M.W.M., and van Zwieten, P.A., "α-Adrenoceptor Agonists and Antagonists", Drugs of the Future, Vol. 9, No. 1, (January, 1984), pp. 41-55; Megens, A.A.H.P., Leysen, J.E., Awouters, F.H.L., and Niemegeers, C.J.E., "Further Validation of *in vivo* and *in vitro* Pharmacological Procedures for Assessing the α₁ and α₂-Selectivity of Test Compounds: (2) α-Adrenoceptor Agonists", European Journal of Pharmacology, Vol. 129 (1986), pp. 57-64; Timmermans, P.B.M.W.M., de Jonge, A., Thoolen, M.J.M.C., Wilffert, B., Batink, H., van Zwieten, P.A., "Quantitative Relationships between α-Adrenergic Activity and Binding Affinity of α-Adrenoceptor Agonists and Antagonists", Journal of Medicinal Chemistry, Vol. 27 (1984) pp. 495-503; van Meel, J.C.A., de Jonge, A., Timmermans, P.B.M.W.M., and van Zwieten, P.A., "Selectivity of Some Alpha Adrenoceptor Agonists for Peripheral Alpha-1 and Alpha-2 Adrenoceptors in the Normotensive Rat", The Journal of Pharmacology and Experimental Therapeutics, Vol. 219, No. 3 (1981), pp. 760-767; Chapleo, C.B., et. al., "Effect of 1,4-Dioxanyl Substitution on the Adrenergic Activity of Some Standard α-Adrenoreceptor Agents", European Journal of Medicinal Chemistry, Vol. 24 (1989), pp. 619-622; Chapleo, C.B., et. al., "Heteroaromatic Analogues of the α₂-Adrenoreceptor Partial Agonist Clonidine", Journal of Medicinal Chemistry, Vol. 32 (1989), pp. 1627-1630; Clare, K.A., Scrutton, M.C., and Thompson, N.T., "Effects of α₂-Adrenoceptor Agonists and of Related Compounds on Aggregation of, and on Adenylate Cyclase Activity in, Human Platelets", British Journal of Pharmacology, Vol. 82 (1984), pp. 467-476; U.S. Patent No. 3,890,319 issued to Danielewicz, Snarey, and Thomas, Jun. 17, 1975; U.S. Patent No. 5,091,528 issued to Gluchowski, Feb. 25, 1992; U.S. Patent No. 5,478,858 issued to Cupps and Bogdan, Dec. 26, 1995; and U.S. Patent No. 5,541,210 issued to Cupps and Bogdan, Jul. 30, 1996.

In the art, 2-amino-2-derivatives have been synthesized according to many different methods. U.S. Patent No. 4,398,028 issued to Neumann, Aug. 9, 1983; Chapleo, C., et. al., "Heteroaromatic Analogues of the α₂-Adrenoreceptor Partial Agonist Clonidine", Journal of Medicinal Chemistry, Vol. 32 (1989) pp. 1627-1630; U.S. Patent No. 5,130,441 issued to Gluchowski, Jul. 14, 1992; U.S. Patent No. 5,478,858 issued to Cupps and Bogdan, Dec. 26, 1995.

For example, the synthesis of clonidine analogs involves the reaction of 2-thiomethyl-2-imidazoline with an aromatic primary amine in the presence of a large excess of pyridine. However, the literature cites very low yields in this reaction. See Chapleo, C., et. al., "Heteroaromatic Analogues of the α₂-Adrenoreceptor Partial Agonist Clonidine", Journal of Medicinal Chemistry, Vol. 32 (1989) pp. 1627-1630.

Alternative syntheses of 2-amino-2-derivatives have been performed. However, of further disadvantage in these syntheses is the time-consuming, costly, multiple steps required by these syntheses, and/or the use of mercuric or other transition metal reagents which can result in the presence of toxic impurities. U.S. Patent No. 4,398,028 issued to Neumann, Aug. 9, 1983; U.S. Patent No. 5,478,858 issued to Cupps and Bogdan, Dec. 26,1995.

Still further, other synthetic preparations of 2-amino-2-derivatives have been performed. U.S. Patent No. 5,130,441 issued to Gluchowski, Jul. 14, 1992. Gluchowski found that yields in the formation of 2-amino-2-imidazolines could be improved over the Chapleo procedure by coupling an aromatic primary amine with an imidazoline sulfonic acid. However, yield improvements in Gluchowski were only moderate. Further, this synthesis required the low yielding preparation of an imidazoline sulfonic acid intermediate.

It is apparent from the art that higher yielding, more economical methods of preparing 2-amino-2-derivatives would be advantageous. It has been surprisingly discovered that the disadvantages of the literature syntheses of these compounds may be overcome by coupling a primary amine or its salts with an acylated 2-thio-substitued-2-imidazoline, -amidine, or -tetrahydropyrimidine intermediate in the presence of a proton source to give the desired 2-amino-2-derivative in one step. Yields in this reaction are significantly higher than those reported in Chapleo. The reaction is also more favorable than the Neumann and Cupps procedures because it overcomes lengthy syntheses and avoids the use of transition metal reagents.

Further, the present invention overcomes the deficiency of the Gluchowski synthesis. The present invention utilizes, not a sulfonic acid, but rather an acylated 2-thio-substitited-2-imidazoline, -amidine, or -tetrahydropyrimidine as the intermediate in the synthesis of 2-amino-2-derivatives. Generally, acylated, 2-thio-substituted-2-imidazolines are known. However, the known syntheses of acylated 2-thiomethyl-2-imidazolines provide low yields. Kohn, H., et. al., "Syntheses and Pharmacological Activity of Substituted Imidazolinethiones and Thioimidazolines", Journal of Medicinal Chemistry, Vol. 20 (1977) pp. 158-160; Kohn, H., et. al., "Syntheses and Spectral Properties of Substituted Imidazolidones and Imidazolines", Journal of Organic Chemistry, Vol. 42 (1977) pp. 941-948. It has been surprisingly discovered that acylated 2-thio-substituted-2-imidazolines, -amidines, and -tetrahydropyrimidines can be prepared in a two-step, one-pot procedure in high yields. This procedure renders the synthesis of acylated 2-thio-substituted-2-derivatives higher yielding, easier, and less time consuming than the procedure in the Kohn reference.

It has therefore now been discovered that 2-amino-2-imidazoline, guanidine, and 2-amino-3,4,5,6-tetrahydropyrimidine derivatives may be conveniently synthesized in high yields by preparing the corresponding acylated 2-thio-substituted-2-derivative in a two-step, one-pot procedure in high yields and by further reacting this isolated derivative with the appropriate amine or its salts in the presence of a proton source. The present process allows for the preparation of 2-amino-2-imidazolines, guanidines, and 2-amino-3,4,5,6-tetrahydropyrimidines under reaction conditions that eliminate the need for lengthy, costly, or multiple low yielding steps, and highly toxic reactants. This process allows for improved yields and product purity and provides additional synthetic flexibility for the preparation of these classes of molecules.

In particular, the preferred processes of the present invention provide a new methodology that is especially suited for the scale-up and manufacture of 2-amino-2-derivatives. The processes utilize commercially-available, low-cost starting materials. The acylated 2-thio-substituted-2-imidazoline, -amidine, or -tetrahydropyrimidine intermediate and the corresponding 2-amino-2-derivative can often be obtained by direct precipitation, thus avoiding the typical extraction and evaporation procedures which are encountered in the literature procedures.

### SUMMARY OF THE INVENTION

The present invention provides a process for making 2-amino-2-imidazoline, guanidine, and 2-amino-3,4,5,6-tetrahydropyrimidine derivatives having a general structure: 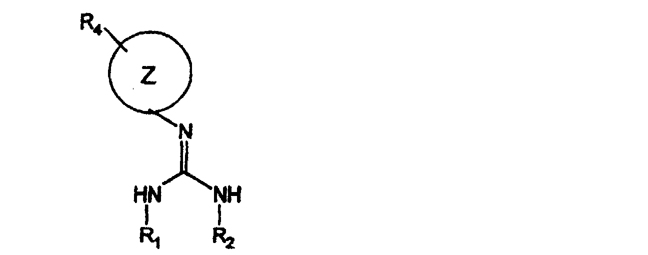 or the tautomers thereof, wherein:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring;
(c) Z is an alkyl or a saturated, unsaturated or aromatic, monocyclic or polycyclic carbocycle or heterocycle containing one or more heteroatoms selected from O, N, or S; and
(d) R₄ is one or more substituents on Z comprising independently hydrogen, alkoxy, alkylthio, alkyl, alkenyl, amino, carboxyl, cyano, halogen, hydroxy, nitro, and thiol;
(e) or a salt or pharmaceutically-acceptable salt thereof;
which comprises the steps of:
(I) preparing an intermediate having the general structure: 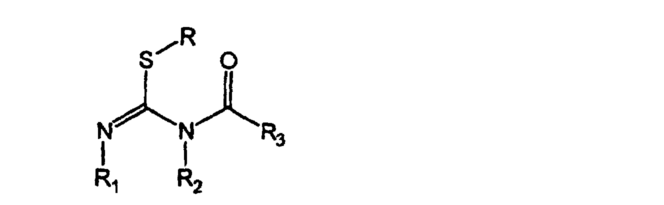 wherein:
   (a) R is selected from the group consisting of methyl, ethyl, and benzyl;
   (b) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
   (c) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring;
   (d) R₃ is -O-R₅ or -R₆;
   (e) R₅ is selected from the group consisting of allyl, methyl, ethyl, benzyl, *tert*-butyl, and phenyl; and
   (f) R₆ is selected from the group consisting of methyl, ethyl, *tert*-butyl, and phenyl;
from a thiourea having the general structure: 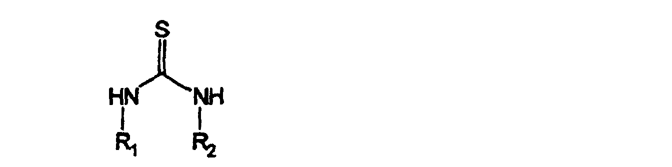 wherein:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring; in a two-step, one-pot reaction by:
   a) alkylating the thiourea using an alkylating agent to form a 2-thio-substituted-2-imidazoline, an amidine, or 2-thio-substituted-3,4,5,6-tetrahydropyrimidine;
   b) acylating the 2-thio-substituted-2-imidazoline, amidine, or 2-thio-substituted-3,4,5,6-tetrahydropyrimidine of step (I)(a) with an acylating agent in the presence of a base; and

(II) coupling the intermediate of step (I) with an amine or its salts of structure: 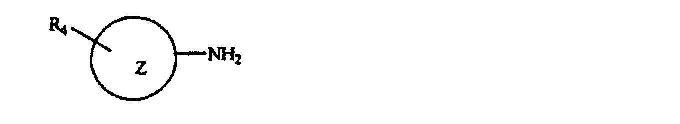 in the presence of an organic acid.

### DETAILED DESCRIPTION OF THE INVENTION

The present invention relates to processes for the manufacture of 2-amino-2-imidazoline, guanidine, and 2-amino-3,4,5,6-tetrahydropyrimidine derivatives. Such 2-amino-2-derivatives are useful for treating various medical disorders, including respiratory disorders, ocular disorders, gastrointestinal disorders, nasal decongestion, hypertension, migraine, disorders associated with sympathetic nervous system activity, and substance abuse. When the compounds made according to these processes are used for treating such disorders, they must be pharmaceutically-acceptable. As used herein, such a "pharmaceutically acceptable" component is one that is suitable for use with humans and/or animals without undue adverse side effects (such as toxicity, irritation, and allergic response) commensurate with a reasonable benefit/risk ratio. Such pharmaceutically-acceptable forms include salts, biohydrolyzable esters and solvates.

The 2-amino-2-derivatives prepared according to the processes of the present invention may also be used as intermediates for preparation of other 2-amino-2-derivatives. That is, the compounds prepared may be further reacted, using known chemistry, to yield other active analogs.

### Definitions and Usage of Terms

The following is a list of definitions for terms used herein:

As used herein, "acylating agent" means a reagent suitable for acylating a nitrogen atom to form a carbamate or an amide, preferably a carbamate. Preferred acylating agents include di-*tert*-butyl dicarbonate, diethylpyrocarbonate, dimethylpyrocarbonate, methyl chloroformate, ethyl chloroformate, benzyl chloroformate, allyl chlorofornate, phenyl chloroformate, acetyl chloride, propionyl chloride, acetic anhydride, propionic anhydride, trimethylacetyl chloride, trimethylacetic anhydride, and benzoyl chloride. More preferred acylating agents are di-*tert*-butyl dicarbonate, dimethylpyrocarbonate, and methyl chloroformate. The most preferred acylating agent is methyl chloroformate.

As used herein, "alkenyl" means a hydrocarbon substituent with one or more double bonds, straight or branched chain, unsubstituted or substituted.

As used herein, "alkoxy" means a substituent having the structure Q-O-, where Q is alkyl or alkenyl.

As used herein, "alkyl" means a saturated hydrocarbon substituent, straight or branched chain, unsubstituted or substituted.

As used herein, "alkylating agent" means a reagent suitable for alkylating a heteroatom such as sulfur. Preferred alkylating agents include methyl iodide, methyl bromide, methyl chloride, dimethyl sulfate, ethyl iodide, ethyl bromide, ethyl chloride, diethyl sulfate, and benzyl bromide. More preferred alkylating agents include methyl iodide, methyl bromide, dimethyl sulfate, ethyl iodide and diethyl sulfate. The most preferred alkylating agents are methyl iodide and dimethyl sulfate.

As used herein, "alkylthio" means a substituent having the structure Q-S-, where Q is alkyl or alkenyl.

As used herein, "base" means a basic reagent which is added to a reaction mixture to facilitate acylation of nitrogen using an acylating agent. Bases include nitrogen bases and inorganic bases. Preferred bases include those which have easily filterable or otherwise removable salts. Specifically, preferred bases include N,N-diisopropylethylamine, triethylamine, trimethylamine, 4-dimethylaminopyridine, pyridine, potassium carbonate, sodium carbonate, potassium bicarbonate, and sodium bicarbonate. The more preferred bases are triethylamine, trimethylamine, and potassium carbonate. The most preferred base is potassium carbonate.

As used herein, "carbocyclic ring" is a saturated, unsaturated, or aromatic, hydrocarbon ring radical. Carbocyclic rings are monocyclic or are fused, bridged, or spiro polycyclic ring systems. Monocyclic rings contain from 3 to 9 atoms, preferably 4 to 7 atoms, and most preferably 5 or 6 atoms. Polycyclic rings contain from 7 to 17 atoms, preferably from 7 to 14 atoms, and most preferably 9 or 10 atoms.

As used herein, "ether solvent" is a solvent which has two alkyl groups bonded to an oxygen, including those in which the alkyl groups and oxygen atom are part of a ring. Preferred ether solvents include diethyl ether, methyl *tert*-butyl ether. tetrahydrofuran, and isopropyl ether. More preferred ether solvents include methyl *tert-*butyl ether and isopropyl ether. The most preferred ether solvent is methyl *tert*-butyl ether.

As used herein, "halocarbon solvents" are solvents that have one or more halogens attached to a carbon chain. Preferred hydrocarbon solvents include dichloromethane, ethylene dichloride, chloroform, and carbon tetrachloride. More preferred are dichloromethane and ethylene dichloride. Even more preferred is ethylene dichloride.

As used herein, "halogen" is a chloro, bromo, fluoro, or iodo atom radical. Bromo, chloro, and fluoro are preferred halogens.

As used herein, "heterocyclic ring" is a saturated, unsaturated, or aromatic, ring radical comprised of carbon atoms and one or more heteroatoms in the ring. Heterocyclic rings are monocyclic or are fused, bridged, or spiro polycyclic ring systems. Monocyclic rings contain from 3 to 9 atoms, preferably 4 to 7 atoms, and most preferably 5 or 6 atoms. Polycyclic rings contain from 7 to 17 atoms, preferably from 7 to 14 atoms, and most preferably 9 or 10 atoms.

As used herein, "methylene" is a -CH₂- radical.

As used herein, "organic acid" is an organic carboxylic acid, such as formic acid, acetic acid, chloroacetic acid, dichloroacetic acid, propionic acid, benzoic acid, maleic acid, fumaric acid, succinic acid, and tartaric acid. Preferred organic acids include acetic acid, propionic acid, and chloroacetic acid. The most preferred organic acid is acetic acid.

As used herein, "polar aprotic solvent" is a solvent that possesses the property of high polarity, yet does not have the ability to donate a proton. Preferred polar aprotic solvents include, acetonitrile, methyl ethyl ketone, N,N-dimethylformamide, N,N-dimethylacetamide, N-methylpyrrolidinone, and methyl sulfoxide. The most preferred polar aprotic solvents are acetonitrile and N,N-dimethylacetamide.

As used herein, "protic solvent" is a solvent that contains a hydrogen atom that is attached to an oxygen or nitrogen atom. Preferred protic solvents include methanol, ethanol, 2-propanol, butanol, *sec*-butanol, and isoamyl alcohol. The most preferred protic solvents are ethanol and methanol.

As defined above and as used herein, substituent groups may themselves be substituted. Such substitution may be with one or more substituents. Such substituents include those listed in C. Hansch and A. Leo, Substituent Constants for Correlation Analysis in Chemistry and Biology (1979), incorporated by reference herein. Preferred substituents include (for example) alkyl, alkenyl, alkoxy, hydroxy, oxo, amino, aminoalkyl (e.g. aminomethyl, etc.), cyano, halogen, alkoxy, alkoxyacyl (e.g.. carboethoxy, etc.), thiol, aryl, cycloalkyl. heteroaryl, heterocycloalkyl (e.g., piperidinyl, morpholinyl, pyrrolidinyl, etc.), imino, thioxo, hydroxyalkyl, aryloxy, arylalkyl, and combinations thereof.

### Compounds Prepared Using the Present Process

The compounds made by the processes of this invention encompass any of a variety of heteroaryl 2-amino-2-imidazolines, guanidines, and 2-amino-3,4,5,6-tetrahydropyrimidines. These compounds are of the following general structure: 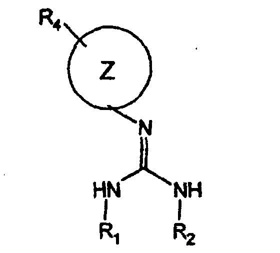 or the tautomers thereof, wherein:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring; and
(c) Z is an alkyl or a saturated, unsaturated or aromatic, monocyclic or polycyclic carbocycle or heterocycle containing one or more heteroatoms selected from O, N, or S; and
(d) R₄ is one or more substituents on Z comprising independently hydrogen, alkoxy, alkylthio, alkyl, alkenyl, amino, carboxyl, cyano, halogen, hydroxy, and thiol;
(e) or a salt or pharmaceutically-acceptable salt thereof.

As used herein, R₁ and R₂ are more preferably methylene groups bonded together either through a single bond or another methylene group to form either a five-membered or a six-membered ring, respectively. Further, it is also more preferable that only R₁ or R₂ are methyl, the other substituent being ethyl. R₁ and R₂ are most preferably methylene groups bonded together through a single bond to form a five-membered ring.

As used herein, Z is more preferably an aromatic monocyclic or polycyclic ring. When Z is monocyclic, Z is preferably a five- or six-membered ring and most preferably a six-membered ring. When Z is polycyclic, Z is preferably a six-membered ring fused with either one or two five- or six-membered rings. When Z is polycyclic, Z is most preferably a six-membered ring fused with a five-membered ring.

As used herein, R₄ is preferably hydrogen, alkoxy, alkylthio, alkyl, alkenyl, amino, carboxyl, cyano, halogen, hydroxy, nitro, or thiol. R₄ is more preferably hydrogen, cyano, alkoxy, alkylthio, amino, C₁ - C₄ alkyl, C₁ - C₄ alkenyl, halogen, or hydroxy. R₄ is most preferably hydrogen, cyano, alkoxy, C₁ - C₄ alkyl, C₁ - C₄ alkenyl, or halogen.

Where the compounds synthesized using the present methods are used as intermediates, groups such as amines, imines, or alcohols may be functionalized through methods well known in the art.

The ordinarily skilled artisan will appreciate that tautomeric forms will exist in certain compounds of the invention. When tautomer A of the molecule is shown, it is understood to include tautomers B and C of that molecule although not specifically depicted. To illustrate:

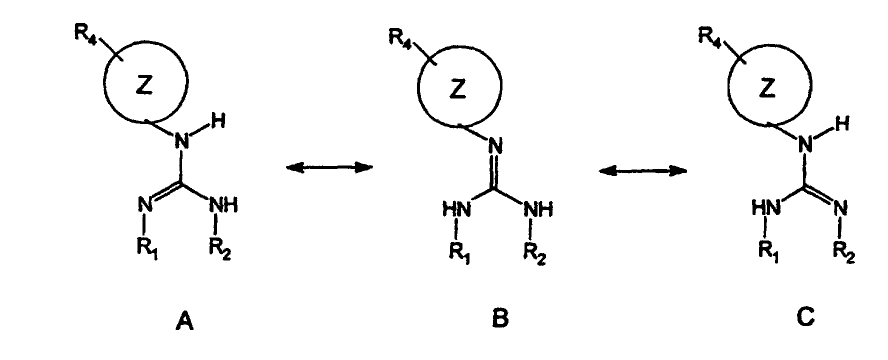

Examples of compounds which may be prepared using the process of the present invention are shown below. These compounds are presented for illustrative purposes only and by no means represent an exhaustive list of possibilities. 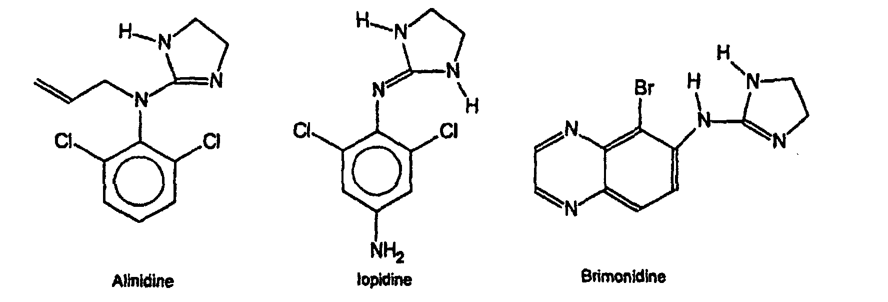 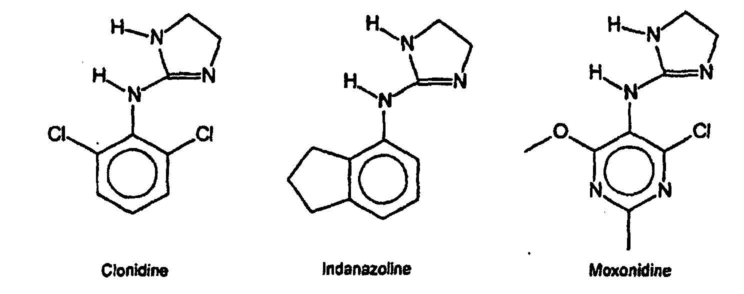 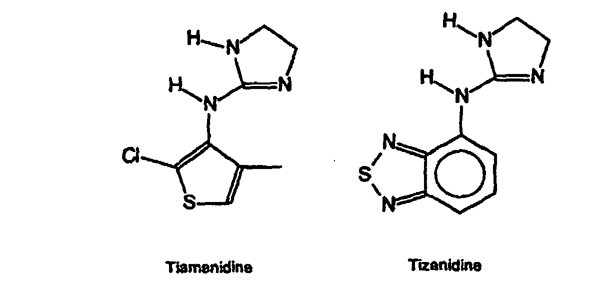 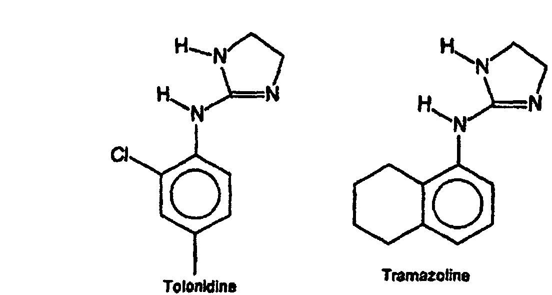 The above molecules are disclosed in the following sources: Alinidine, disclosed in U.S. Patent No. 3,708,485 (1973) to Boehringer, Ing.; Iopidine, disclosed in U.S. Patent No. 4,517,199 (1985) to Alcon; Brimonidine, disclosed in German Patent No. 2,538,620 to Pfizer; Clonidine, disclosed in U.S. Patent No. 3,202,660 (1965) to Boehringer, Ing.; Indanazoline, disclosed in U.S. Patent No. 3,882,229 to Nordmark; Moxonidine, U.S. Patent No. 4,323,570 (1982) to Beiersdorf Aktiengesellschaft; Tiamendine, disclosed in U.S. Patent No. 3,758,476 (1973) to Hoechst; Tizanidine, disclosed in U.S. Patent No. 3,843,668 (1974) to Wander-Sandoz; Tolonidine, disclosed in U.S. Patent No. 3,236,857 (1966) to Boehringer, Ing.; Tramazoline, disclosed in German Patent No. 1,191,381 (1965) to Thomae.

### Methods of Manufacture

Generally, the processes of the present invention comprise the novel synthesis of an acylated 2-thio-substituted-2-imidazoline, -amidine, or -3,4,5,6-tetrahydropyrimidine intermediate (hereinafter described as the "acylated intermediate") followed by coupling of the acylated intermediate with an appropriate amine or its salts in the presence of an organic acid. The acylated intermediate used in the synthesis is conveniently prepared in a novel two-step, one-pot reaction from the appropriate thiourea in high yields.

This process is illustrated by the following general scheme: 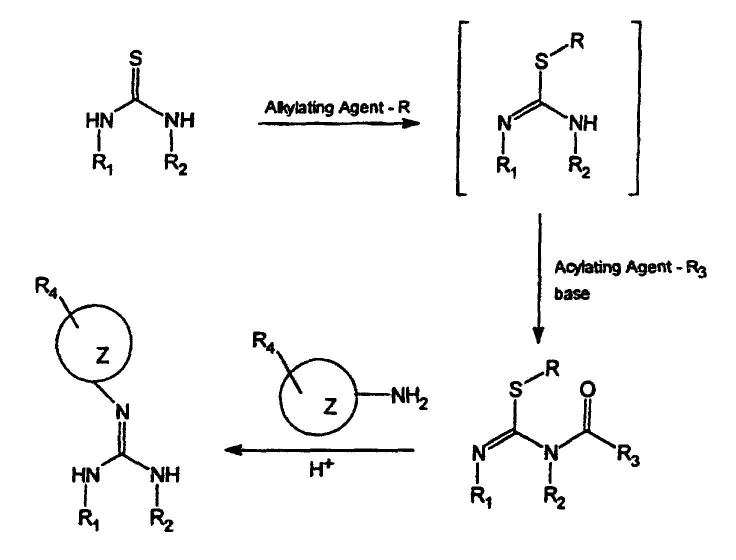 In the above general scheme:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring; and

R₁ and R₂ are more preferably methylene groups bonded together either through a single bond or another methylene group to form either a five-membered or a six-membered ring, respectively. R₁ and R₂ are most preferably methylene groups bonded together through a single bond to form a five-membered ring.

In the above general scheme, R is an alkyl or aromatic substituent derived from the alkylating agent used in the process. R is preferably a methyl, ethyl, or benzyl radical. R is most preferably a methyl radical.

In the above general scheme, R₃ is derived from the acylating agent used in the process. R₃ may be -O-R₅, or -R₆, wherein R₅ and R₆ are also derived from the acylating agent used in the process. R₃ is preferably -O-R₅, R₅ is preferably an allyl, methyl, ethyl, benzyl, *tert*-butyl, or phenyl radical. R₅ is most preferably a methyl radical. R₆ is preferably a methyl, ethyl, *tert*-butyl, or phenyl radical.

In the above general scheme, Z is an alkyl or a saturated, unsaturated or aromatic, monocyclic or polycyclic carbocycle or heterocycle containing one or more heteroatoms selected from O, N, or S. Z is preferably an aromatic monocyclic or polycyclic ring. When Z is monocyclic, Z is preferably a five- or six-membered ring and most preferably a six-membered ring. When Z is polycyclic, Z is preferably a six-membered ring fused with either one or two five- or six-membered rings. When Z is polycyclic, Z is most preferably a six-membered ring fused with a five-membered ring.

In the above general scheme, R₄ is one or more substituents on Z comprising independently hydrogen, alkoxy, alkylthio, alkyl, alkenyl, amino, carboxyl, cyano, halogen, hydroxy, nitro, and thiol. R₄ is more preferably hydrogen, cyano, alkoxy, alkylthio, amino, C₁ - C₄ alkyl, C₁ - C₄ alkenyl, halogen, or hydroxy. R₄ is most preferably hydrogen, cyano, alkoxy, C₁ - C₄ alkyl, C₁ - C₄ alkenyl, or halogen.

In the above general scheme, a thiourea is reacted with an alkylating agent in a solvent that will allow the alkylation reaction to proceed. More preferred alkylating agents include methyl iodide, methyl bromide, dimethyl sulfate, ethyl iodide and diethyl sulfate. The most preferred alkylating agents are methyl iodide and dimethyl sulfate. Preferred solvents include ester solvents (such as, for example, butyl acetate, ethyl acetate, or methyl acetate, preferably ethyl acetate), ether solvents, protic solvents, and polar aprotic solvents. More preferred solvents include ether solvents, protic solvents and polar aprotic solvents. The most preferred solvents are protic solvents. The most preferred solvent is ethanol. The mixture is allowed to proceed at a temperature preferably between about 0 °C and about 150 °C, more preferably between ambient temperature and about 100 °C, and most preferably between about 30 °C and about 70 °C.

The thio-substituted compound so obtained can be isolated by methods obvious to those who are skilled in the art, such as using methods including extraction, solvent evaporation, distillation, or crystallization procedures. Most preferably, the 2-thio-2-substituted derivative is further reacted in the same vessel and in the same solvent without isolation. Prior to further reaction of the 2-thio-2-substituted derivative, the reaction mixture is preferably cooled from about -10 °C to about 75 °C, more preferably cooled from about 0 °C to about 40 °C, and most preferably cooled to ambient temperature.

The 2-thio-2-substituted derivative is then reacted with an acylating agent in the presence of a base, in any solvent that allows the reaction to proceed. Preferred acylating agents include di-*tert*-butyl dicarbonate, diethylpyrocarbonate, dimethylpyrocarbonate, methyl chloroformate, ethyl chlorofonnate, allyl chloroformate, phenyl chloroformate, acetyl chloride, propionyl chloride, acetic anhydride, propionic anhydride, trimethylacetyl chloride, trimethylacetic anhydride, and benzoyl chloride. More preferred acylating agents are di-*tert*-butyl dicarbonate, dimethylpyrocarbonate, methyl chloroformate, and acetic anhydride. The most preferred acylating agent is methyl chloroformate. Preferred bases include those which have easily filterable or otherwise removable salts. Specifically, the more preferred bases are triethylamine and potassium carbonate. The most preferred base is potassium carbonate. Preferred solvents include ester solvents (such as, for example, butyl acetate, ethyl acetate, or methyl acetate, preferably ethyl acetate), ether solvents, protic solvents, and polar aprotic solvents. More preferred solvents include ether solvents, protic solvents (particularly ethanol and isopropanol) and polar aprotic solvents (particularly N,N-dunethylacetamide). The most preferred solvents are protic solvents. The most preferred solvent is ethanol. The base is preferably added to the reaction mixture first, followed by the acylating agent, maintaining the temperature of the mixture preferably between about 0 °C to about 50 °C, more preferably between about 20 °C to about 35 °C. The reaction is allowed to proceed at a temperature preferably between about 20 °C to about 60 °C, more preferably between about 40 °C to about 55 °C.

Upon completion of the reaction, the acylated intermediate so obtained can be isolated by methods known to those who are skilled in the art, such as methods including extraction, solvent evaporation, distillation or crystallization procedures. More preferably, the reaction mixture is filtered to remove the by-product salts, at a temperature between about 30 °C to about 70 °C, more preferably between about 50 °C to about 60 °C. The by-product salts are then preferably rinsed with ester solvents (such as, for example, butyl acetate, ethyl acetate, or methyl acetate, preferably ethyl acetate), protic solvents, or polar aprotic solvents, more preferably with a protic or ester solvent. After filtration, the acylated intermediate so obtained can be isolated by methods known to those who are skilled in the art, such as using methods including extraction, solvent evaporation, distillation, or crystallization procedures. Preferably, the product is isolated as a solid, by cooling the filtrate to a temperature from about -30 °C to ambient temperature, more preferably from about -20 °C to about 0 °C. The solid so obtained is filtered and rinsed with a protic or ester solvent that has been pre-cooled to between about -30 °C to ambient temperature, more preferably between about -20 °C to about 20 °C. The solid is preferably dried by methods known to those who are skilled in the art.

The acylated intermediate can then be further reacted with the appropriate amine or its salts in a protic solvent or a polar aprotic solvent or mixtures thereof, in the presence of an organic acid. The acylated intermediate may also be further reacted with the appropriate amine or its salts in a solution of the organic acid alone. Preferred acids include formic acid, acetic acid, chloroacetic acid, dichloroacetic acid, propionic acid, benzoic acid, maleic acid, fumaric acid, succinic acid, or tartaric acid. The preferred protic solvents include methanol and ethanol. The most preferred polar aprotic solvent is acetonitrile. The reaction is preferably carried out at a temperature between ambient temperature and about 150 °C, more preferably between about 40 °C to about 100 °C, and even more preferably between about 55 °C to about 80 °C. In some cases, wherein R₃ is not readily removable, it may be necessary to add an inorganic acid such as HCl or HBr, additional amounts of an organic acid, or a more protic solvent, and/or apply increased heating to the reaction mixture to facilitate cleavage of the acyl group. Those skilled in the art will recognize that hydrolysis of this acyl group may also be achieved under basic conditions as well. Upon completion of the reaction, the 2-amino-2-derivative so obtained can be isolated by methods known to those who are skilled in the art, such as methods including extraction, solvent evaporation, distillation or crystallization procedures. Those skilled in the art will also recognize that various acids may be added in the final stages of the process to form various salt forms which may facilitate isolation and handling.

The following non-limiting examples illustrate the processes of the present invention:

### Example 1

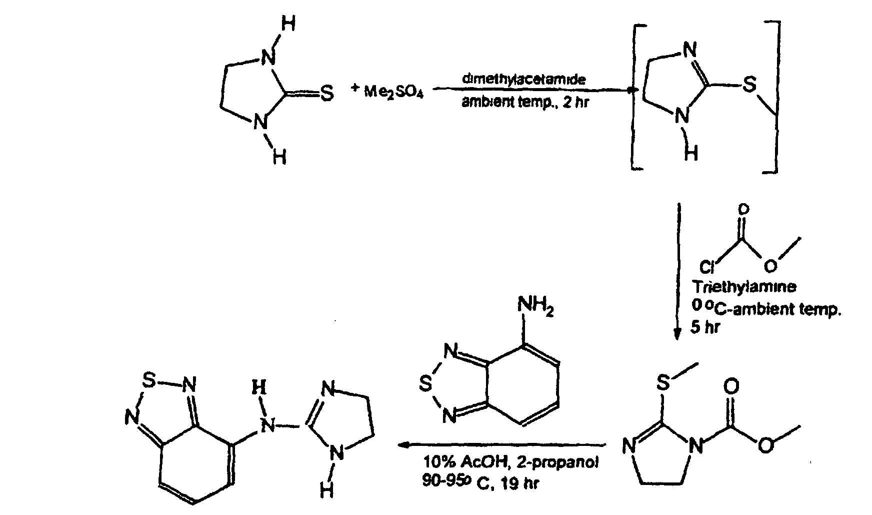

a. 2-Thiomethyl-2-Imidazoline:
   2-Imidazolidinethione (150 grams, 1.5 mol) and N,N-dimethylacetamide (1.1 L) are combined together in a round-bottom flask. To this is added dimethyl sulfate (213 grams, 1.7 mol) at ambient temperature. This reaction is stirred for two hours.
b. N-Carbomethoxy-2-thiomethyl-2-imidazoline:
   The reaction mixture of step (a) is cooled in an ice bath and to this stirred solution is added triethylamine (376 grams, 3.7 mol) in a dropwise manner. To this mixture is added methyl chloroformate (166.7 grams, 1.8 mol) in a dropwise manner. After completion of addition, the reaction mixture is allowed to warm to ambient temperature. After 5 hours of stirring, the mixture is poured into cold water (3 L). The product is extracted into ethyl acetate (4 x 2.5 L). The combined extracts are washed with cold water, brine, dried over sodium sulfate, and concentrated under reduced pressure. Drying the residue under vacuum affords the desired N-carbomethoxy-2-thiomethyl-2-imidazoline.
c. 4-(2-Imidazolinylamino)-1,3,2-benzothiadiazole, acetate salt:
   N-Carbomethoxy-2-thiomethyl-2-imidazoline (19.2 grams, 11 mmol) and 4-amino-2,1,3-benzothiadiazole (11.1 grams, 73 mmol) are dissolved in a 10% solution of glacial acetic acid in 2-propanol (500 mL). The resulting solution is heated near reflux (90-95 °C) for 19 hours. The mixture is concentrated under reduced pressure, redissolved in 2-propanol and reprecipitated to yield 4-(2-imidazolinylamino)-1,3,2-benzothiadiazole, acetate salt.

### Example 2

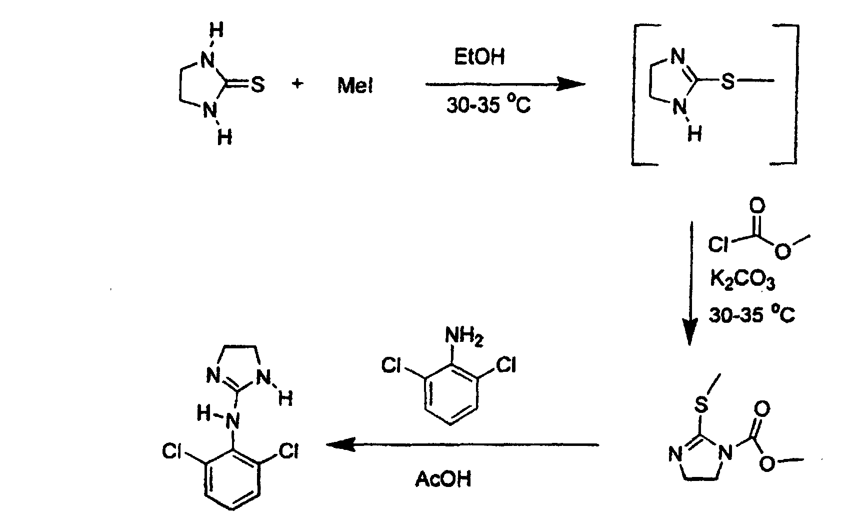

a. 2-Thiomethyl-2-imidazoline:
   2-Imidazolidinethione (50 grams) and absolute ethanol (400 mL) are combined while stirring. Methyl iodide (43 mL, 1.4 eq.) is rapidly added to the stirring mixture. The reaction mixture is then warmed to 35 °C until the formation of 2-thiomethyl-2-imidazoline is complete.
b. N-Carbomethoxy-2-thiomethyl-2-imidazoline:
   Potassium carbonate (101 grams) is added to the mixture in step (a) above, followed by addition of methyl chloroformate (42 mL) while stirring. After 45 minutes, the reaction mixture is heated to 55 °C and the insoluble salts are filtered off. These salts are washed with absolute ethanol. The filtrate (and ethanol wash) are cooled to -20 °C and the final product is isolated by filtration. The final product is washed with cold (-20 °C) absolute ethanol. The product is dried overnight under vacuum at room temperature, giving N-carbomethoxy-2-thiomethyl-2-imidazoline.
c. 2-[(2,6-Dichlorophenyl)amino]-2-imidazoline, acetate salt:
   2,6-Dichloroaniline (2 g) and N-carbomethoxy-2-thiomethyl-2-imidazoline (2.68 g) are dissolved in glacial acetic acid (40 mL), and the reaction is stirred at 65 to 75°C until coupling step is complete. The reaction mixture is diluted with 70% methanol-water (40 ml), refluxed until deprotection is complete, and then concentrated under reduced pressure to furnish an oily residue. Addition of ethyl acetate to the oily residue precipitates the impurities which are separated by filtration. Concentration of the filtrate provides 2-[(2.6-dichlorophenyl)amino]-2-imidazoline as an acetate salt.

### Example 3

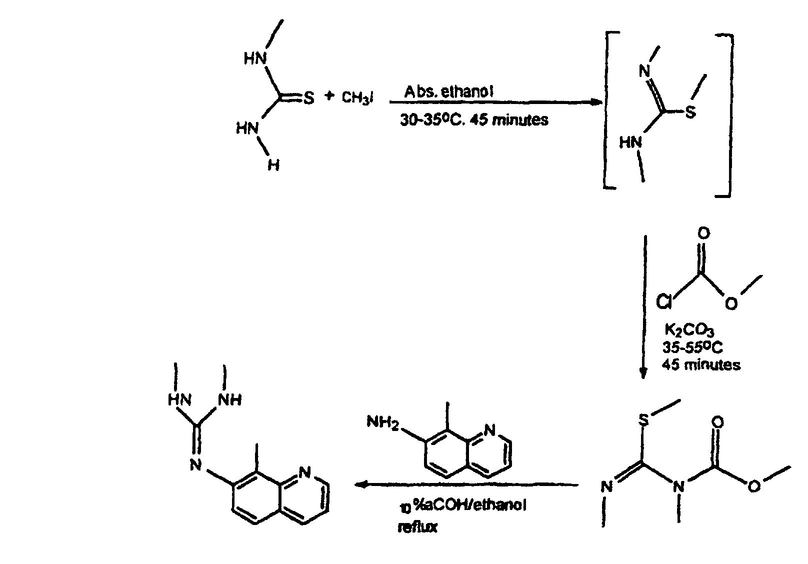

a N,N-Dimethyl-(2-thiomethyl)amidine:
   To 1,3 - dimethyl-2-thiourea (50 grams, 480 mmol) is added absolute ethanol (400 mL) with stirring. Iodomethane (43 mL, 690 mmol) is added rapidly. The reaction mixture is warned to 30-35 °C and is stirred until the formation of N,N'-dimethyl-(2-thiomethyl)amidine is complete.
b. N,N'-Dimethyl-(N-methoxycarbonyl-2-thiomethyl)amidine:
   Potassium carbonate (101 grams) is added to the mixture in step (a) above. Methyl chloroformate (42 mL, 540 mmol) is then added. After 1 hour, the reaction mixture is heated to 55 °C and the insoluble salts are filtered. The salts are washed with ethanol (100 mL). The filtrate (and ethanol wash) are cooled to -20 °C and the final product is isolated by filtration. The final product is washed with 100 mL cold (-20 °C) absolute ethanol. The 2-thiomethylamidine is dried overnight under vacuum at ambient temperature.
c. N.N'-Dimethyl-N"-(8-methylquinolin-7-yl)guanidine. acetate salt:
   The intermediate prepared in step (b) above is combined with 0.7 equivalents of 7-amino-8-methylquinoline (prepared in U.S. Patent No. 5,576,437 issued to Cupps and Bogdan, Nov. 19, 1996 (incorporated herein by reference) in a 10% solution of glacial acetic acid in ethanol (2 L). The mixture is heated to reflux and after the starting amine is consumed, the mixture is decolorized with deactivated carbon. The product is cooled to ambient temperature, filtered, dried, and recrystallized from acetonitrile and water. Upon drying under high vacuum, N,N'-Dimethyl-N"-(8-methylquinolin-7-yl)guanidine, acetate salt is obtained.

### Example 4

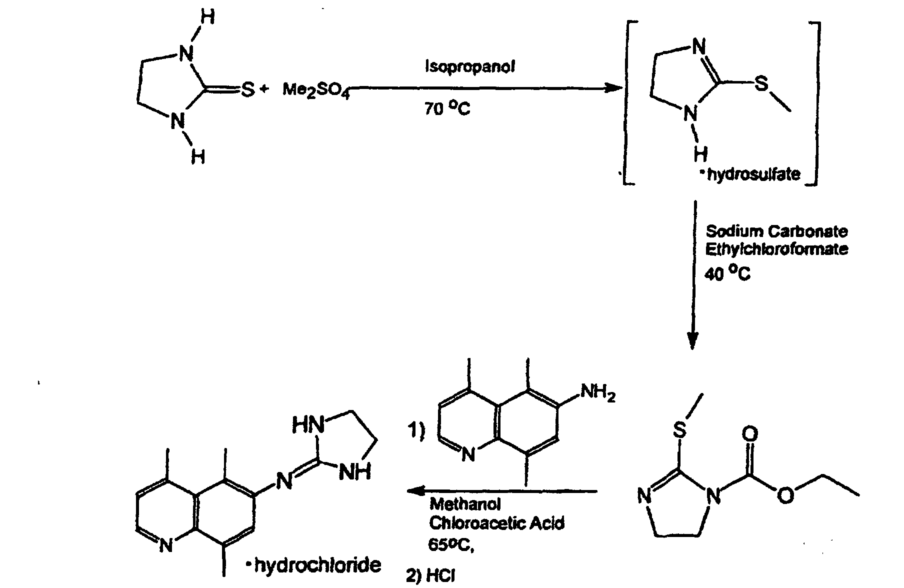

a. 2-Thiomethyl-2-imidazoline:
   Dimethylsulfate (111 mL) is slowly added to a stirred solution of 2-imidazolidinethione (120 g) in isopropanol (750 mL) at ambient temperature. The reaction mixture is heated to 70 °C until the formation of 2-thiomethyl-2-imidazoline hydrosulfate is complete.
b. N-Carboethoxy-2-thiomethyl-2-imidazoline:
   The reaction mixture of step (a) is allowed to cool to ambient temperature, whereupon sodium carbonate (249 g) is added, followed by the addition of ethylchlorofortnate (168 mL). The reaction mixture is stirred at 40 °C until complete, whereupon the reaction mixture is heated to 55 °C and the hot mixture is filtered to remove the insoluble salts. These salts are washed with cold isopropanol. The filtrate (and wash solution) is cooled to -20 °C and stirred for 2 hours. The solid obtained is filtered off and washed with cold water and then cold absolute ethanol. The product is dried under vacuum at room temperature to provide N-Carboethoxy-2-thiomethyl-2-imidazoline as a solid.
c. 6-(2-Imidazolinylamino)-4,5,8-trimethylquinoline, hydrochloride salt:
   6-Amino-4,5,8-trimethylquinoline (115.5 g) (as prepared in Example 2 of copending U.S. Patent application serial No. 08/169,343) and N-carboethoxy-2-thiomethyl-2-imidazoline (140 g), is dissolved in 10% chloroacetic acid in methanol (2.8 L, w/w) and stirred at 65 °C until complete. The reaction mixture is cooled to room temperature and HCl gas is added. The reaction mixture is stirred for 2 hours and then cooled to -20 °C and stirred until the product completely precipitates. The crude product so obtained is filtered, recrystallized from ethanol/water and dried (vacuum, 40 °C) to provide the desired, purified 6-(2-imidazolinylamino)-4,5,8-trimethylquinoline, hydrochloride salt.

### Example 5

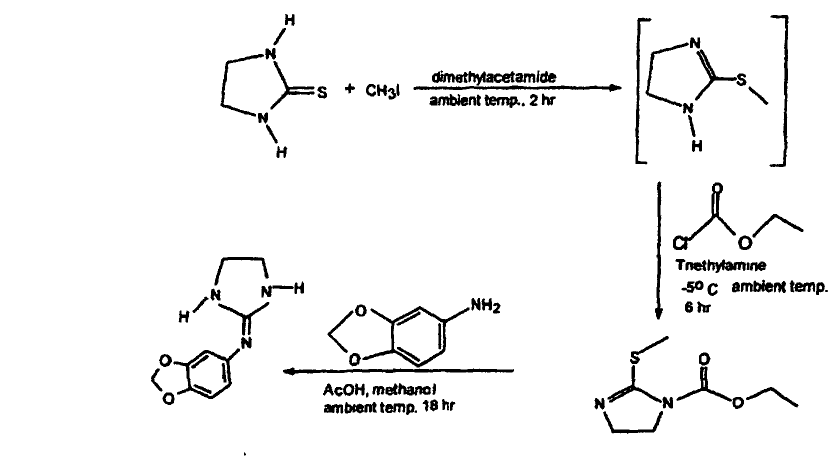

a. 2-Thiomethyl-2-Imidazoline:
   2-Imidazolidinethione (68 grams, 670 mmol) and N,N-dimethylacetamide (700 mL) are combined together in a round-bottom flask. To this is added iodomethane (50 mL, 810 mmol) at ambient temperature. This reaction is allowed to stir for two hours.
b. N-Carboethoxy-2-thiomethyl-2-imidazoline:
   The reaction mixture is cooled in an ice bath and to this stirred solution is added triethylamine (250 mL, 1.8 mol) in a dropwise manner. To this mixture is added ethyl chloroformate (81 mL, 850 mmol) in a dropwise manner at -5 °C. Five minutes after completion of addition, the reaction mixture is allowed to warm to ambient temperature and is then stirred for 6 hours. The reaction is poured into ice cold water (4 L). The product is extracted into ethyl acetate (4 x 2.5 L). The combined extracts are washed with cold water (3 x 2 L), brine (2 L), dried over sodium sulfate, and concentrated under reduced pressure to give an oily residue. Drying the residue under vacuum affords the desired N-carboethoxy-2-thiomethyl-2-imidazoline.
c. 2-(1',3'-Benzodioxolyl-5'-amino)imidazoline:
   N-Carboethoxy-2-thiomethyl-2-imidazoline (22.5 grams, 104 mmol) is dissolved in methanol (1 L) and to this is added glacial acetic acid (12 mL, 208 mmol). The reaction mixture is allowed to stir for 10 minutes. To this solution is added 3,4-(methylenedioxy)aniline (14.3 grams, 104 mmol) and the reaction is allowed to stir at ambient temperature until the reaction is complete. The solvent is removed under reduced pressure. The crude product is extracted into ethyl acetate, dried, and evaporated to give 2-(1',3'-benzodioxolyl-5'-amino)imidazoline.

### Example 6

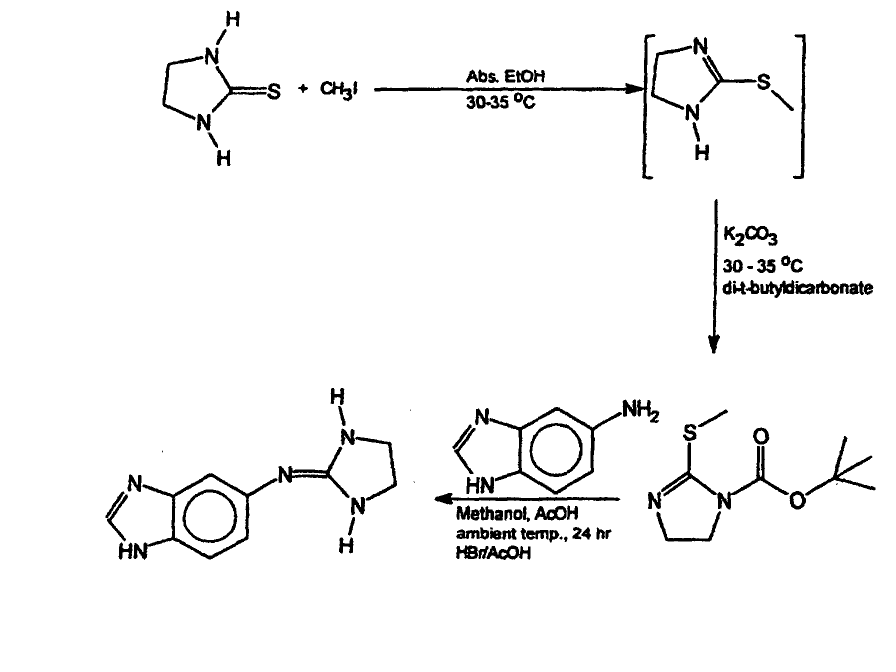

a. 2-Thiomethyl-2-imidazoline:
   2-Imidazolidinethione (10.2 grams, 100 mmol) and dichloromethane (400 ml) are combined in a round-bottom flask equipped with a reflux condenser, with stirring. Iodomethane (8.7 mL, 140 mmol) is rapidly added. The reaction mixture is warmed to 30 °C - 35 °C until the formation of 2-thiomethyl-2-imidazoline is complete.
b. *N*-*t*-Butoxycarbonyl-2-thiomethyl-2-imidazoline:
   The reaction mixture of step (a) above is allowed to cool to ambient temperature. Triethylamine is then added to the stirring mixture (14 mL). To this solution at room temperature is added 4-dimethylaminopyridine (12.2 grams, 100 mmol) and then di-*tert*-butyldicarbonate (65.4 grams, 300 mmol). The reaction is allowed to stir for 6 hours. The solvent is removed under reduced pressure leaving a solid which is further dried under vacuum. The crude material is extracted into ethyl acetate, washed with water, dried, and evaporated to give the pure *N-t*-butoxycarbonyl-2-thiomethyl-2-imidazoline.
c. 5-(2-Imidazolinylamino)-benzimidazole, hydrobromide salt:
   5-Aminobenzimidazole (5 grams, 38 mmol) and *N*-*t*-butoxycarbonyl-2-thiomethyl-2-imidazoline (9.4 grams, 42 mmol) are dissolved in 10% acetic acid in methanol (400 mL) and are stirred for 24 hours at ambient temperature. To this solution is added 30% HBr/AcOH (100 mL) and the reaction is stirred for an additional 4 hours. The resulting solution is concentrated under reduced pressure, redissolved in methanol and recrystallized from methanol/diethyl ether to yield the desired product as the hydrobromide salt.

### Example 7

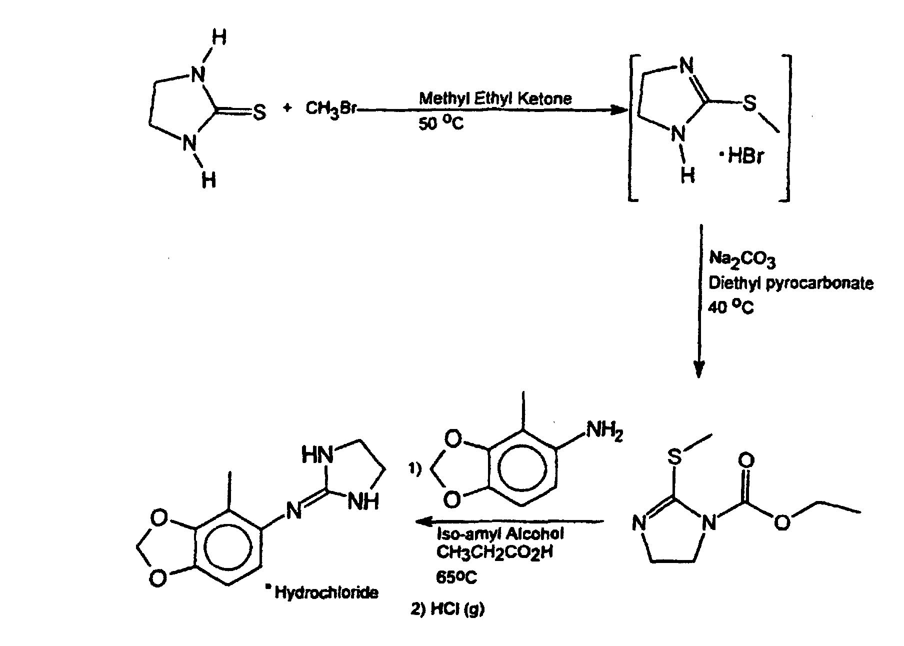

a. 2-Thiomethyl-2-imidazoline:
   In a pressure reactor, methyl bromide (32 g) is slowly added to a solution of 2-imidazolidinethione (17 g) in methyl ethyl ketone (175 mL) with stirring at ambient temperature. The reaction mixture is heated to 65°C under pressure, until the formation of 2-methylthio-2-imidazoline hydrobromide is complete.
b. N-Carboethoxy-2-thiomethyl-2-imidazoline:
   The reaction mixture of step (a) is allowed to cool to ambient temperature and the excess methyl bromide is released and trapped. To this mixture is added sodium carbonate (26.5 g), followed by the addition of diethyl pyrocarbonate (42 mL). The reaction mixture is stirred at 40 °C until complete, whereupon the reaction mixture is heated to 55 °C and the hot solution is filtered to remove the insoluble salts. These salts are washed with cold absolute ethanol. The filtrate (and ethanol wash) is cooled to -20 °C and stirred for 2 hours. The solid obtained is filtered, and washed with cold water and then cold absolute ethanol. The product is dried under vacuum at room temperature to provide N-carboethoxy-2-thiomethyl-2-imidazoline as a solid.
c. 5-(2-Imidazolinylamino)-4-methyl-1,3-benzodioxole, hydrochloride salt:
   5-Amino-4-methyl-1,3-benzodioxole (13.25 g) (as prepared in application serial No. 08/478.708) and N-carboethoxy-2-thiomethyl-2-imidazoline (20 g) are dissolved in 10% propionic acid in isoamyl alcohol (325 mL, w/w) and the reaction mixture is stirred at 65 °C until complete. The reaction mixture is cooled to room temperature and HCl gas (13 g) is slowly added. The mixture is stirred for an additional 2 hours, whereupon it is then cooled to -20 °C and stirred until the product precipitates. The crude product obtained is filtered, recrystallized from methanol/diethyl ether and dried (vacuum, 40 °C) to provide the desired, purified 5-(2-imidazolinylamino)-4-methyl-1,3-benzodioxole, hydrochloride salt.

### Example 8

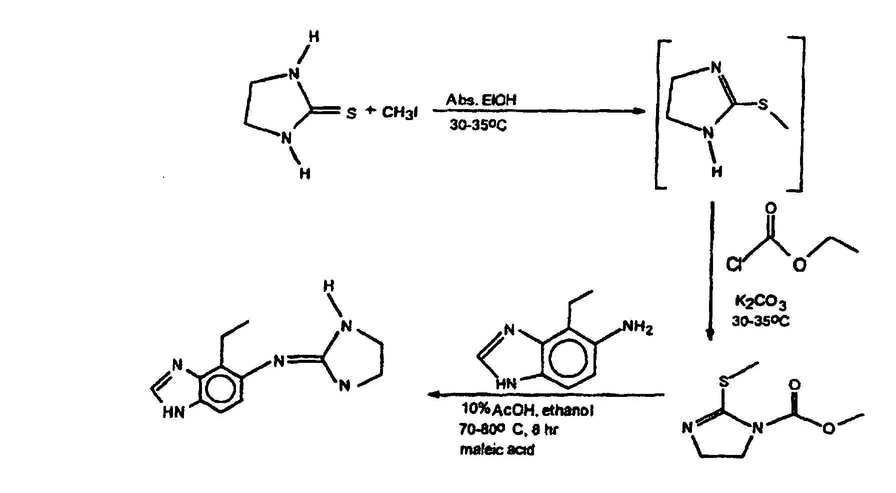

a. 2-Thiomethyl-2-imidazoline:
   2-Imidazolidinethione (50 grams) and absolute ethanol (400 mL) are combined while stirring. Methyl iodide (43 mL, 1.4 Eq) is rapidly added to the stirring mixture. The reaction mixture is then warmed to 35 °C until the formation of 2-thiomethyl-2-imidazoline is complete.
b. N-Carbomethoxy-2-thiomethyl-2-imidazoline:
   Potassium carbonate (101 grams) is added to the mixture in step (a) above, followed by addition of methyl chloroformate (42 mL) while stirring. After 45 minutes, the reaction mixture is heated to 55 °C and the insoluble salts are filtered off. These salts are washed with absolute ethanol. The filtrate (and ethanol wash) are cooled to -20 °C and the final product is isolated on a filter. The final product is washed with cold (-20 °C) absolute ethanol. The product is dried overnight under vacuum at room temperature, giving N-carbomethoxy-2-thiomethyl-2-imidazoline.
c. 4-Ethyl-5-(2-imidazolinylamino)benzimidazole, maleate salt:
   The N-Carbomethoxy-2-thiomethyl-2-imidazoline (23.8 grams, 140 mmol) is combined with 5-amino-4-ethylbenzunidazole (20 grams, 124 mmol) (prepared by deprotecting *tert*-butoxycarbonyl protecting group of intermediate prepared in U.S. Patent No. 5,478,858 issued to Cupps and Bogdan, Dec. 26, 1995 (incorporated herein by reference), under standard deprotection conditions known in the art) and a 10% solution of acetic acid in ethanol (500 mL) in a flask equipped with a reflux condenser. This mixture is stirred for 1 hour. The mixture is then heated at 65 °C for 12 hours. At this time, the reaction is cooled to ambient temperature and maleic acid (48 grams, 410 mmol) is added. The resulting mixture is stirred for two hours and then is cooled to 0 °C. The mixture is stirred until the product completely precipitates (approximately 1 hour), whereupon the mixture is filtered. The crude product is washed with cold ethanol and then recrystallized from acetonitrile/water to give 4-ethyl-5-(2-imidazolinylamino)benzimidazole, maleate salt.

### Example 9

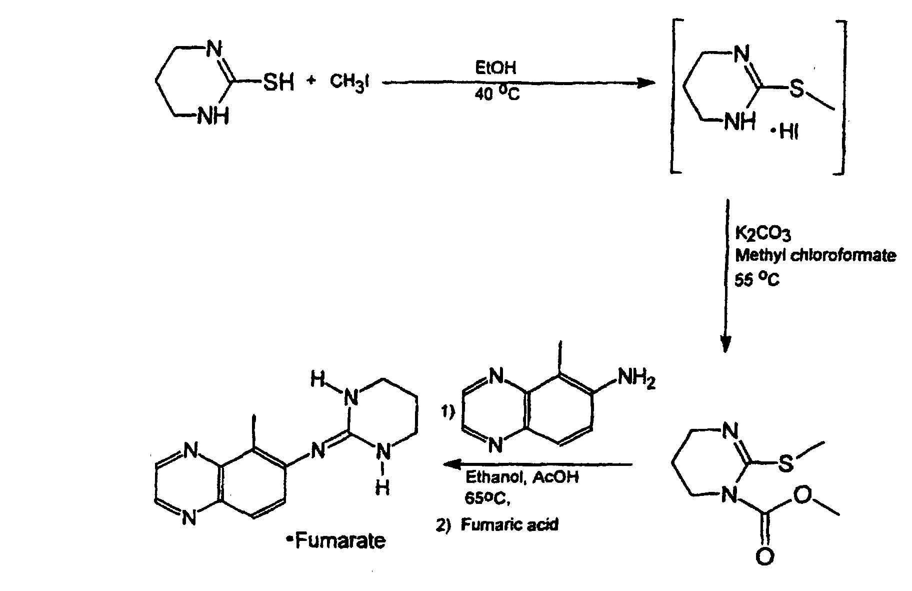

a. 2-Thiomethyl-3,4,5,6-tetrahydropyrimidine:
   Methyl iodide (75 mL) is slowly added to a stirred solution of 3,4,5,6-tetrahydro-2-pyrimidine thiol (100 g) in ethanol (600 mL) at ambient temperature. The reaction mixture is heated to 40 °C until the formation of 2-thiomethyl-3,4,5,6-tetrahydropyrimidine hydroiodide is complete.
b. N-3-Carbomethoxy-2-thiomethyl-4,5,6-tetrahydropyrimidine:
   The mixture in step (a) above is cooled to ambient temperature and potassium carbonate (178 g) is added, followed by addition of methyl chloroformate (73.2 mL) while stirring. The reaction mixture is stirred at 40 °C until complete, whereupon the reaction mixture is heated to 55 °C and the hot solution is filtered to remove the insoluble salts. These salts are washed with cold absolute ethanol. The filtrate (and ethanol wash) is cooled to -20 °C and stirred for 2 hours. The solid obtained is filtered, and washed with cold water and then cold absolute ethanol. The product is dried under vacuum at room temperature to provide N-3-carbomethoxy-2-thiomethyl-4,5,6-tetrahydropyrimidine.
c. 2-(5-Methyl-6-quinoxalinylamino)3,4,5,6-tetrahydropyrimidine:
   6-Amino-5-methylquinoxaline (73.8 g) (as prepared in copending U.S. Patent application serial No.08/478,708) and N-3-carbomethoxy-2-thiomethyl-4,5,6-tetrahydropyrimidine (113.5 g) are dissolved in 10% acetic acid in ethanol (1.1 L) and stirred at 65 °C until complete. The reaction mixture is cooled to ambient temperature and fumaric acid is added (189 g). The mixture is stirred for 2 hours and then cooled to -20 °C and stirred until the product has completely precipitatated. The crude product so obtained is recrystallized from acetonitrile/water to provide the desired, purified 2-(5-Methyl-6-quinoxalinyiamino)3,4,5,6-tetrahydropyrimidine, fumarate salt.

### Example 10

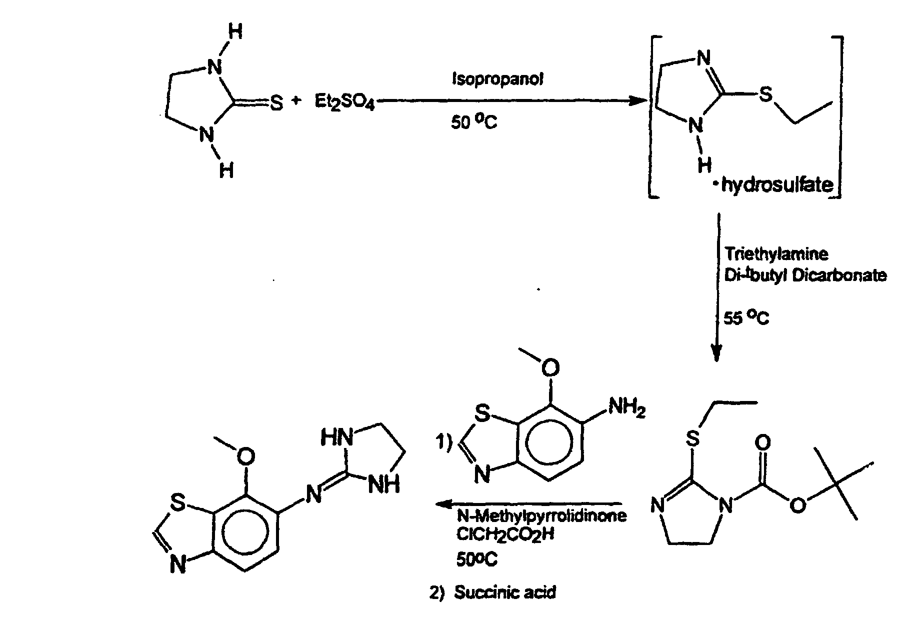

a. 2-Thioethyl-2-imidazoline hydrosulfate:
   Diethyl sulfate (47.75 mL) is slowly added to a solution of 2-imidazolidinethione (30 g) in isopropanol (250 mL) with stirring at ambient temperature. The reaction mixture is heated to 50 °C until the formation of 2-thioethyl-2-imidazoline hydrosulfate is complete.
b. *N-tert*-butoxycarbonyl-2-thioethyl-2-imidazoline:
   The reaction mixture of step (a) is allowed to cool to ambient temperature, whereupon triethylamine (105 mL) and then di-*tert*-butyldicarbonate (74.25 mL) are added. The reaction mixture is heated to 55 °C and stirred until complete. The reaction mixture is then filtered hot, removing the insoluble salts. These salts are washed with cold isopropanol. The filtrate (and isopropanol wash) is cooled to -20 °C and and stirred for 2 hours. The solid obtained is filtered and washed with water and cold absolute ethanol. The product is dried under vacuum at room temperature to provide *N*-*tert*-butoxycarbonyl-2-thioethyl-2-imidazoline as a solid.
c. 5-(2-Imidazolinylamino)-4-methoxybenzothiazole, succinate salt:
   5-Amino-4-methoxybenzothiazole (18 g) (as prepared in Example 5 of copending U.S. Patent application serial No. 60/031,756) and *N*-*tert*-butoxycarbonyl-2-thioethyl-2-imidazoline (32.5 g) are dissolved in 10% chloroacetic acid in N-methylpyrrolidinone (390 mL, w/w). The mixture is then stirred at 50 °C until the reaction is complete. The mixture is cooled to ambient temperature and succinic acid (47.5 g) is added, and the mixture is stirred for an additional 4 hours. The resulting solution is cooled to -20 °C and stirred until the product completely precipitates. The crude product is then filtered and recrystallized from ethanol/water to provide the desired, purified salt of 5-(2-imidazolinylamino)-4-methoxybenzothiazole.

### Example 11

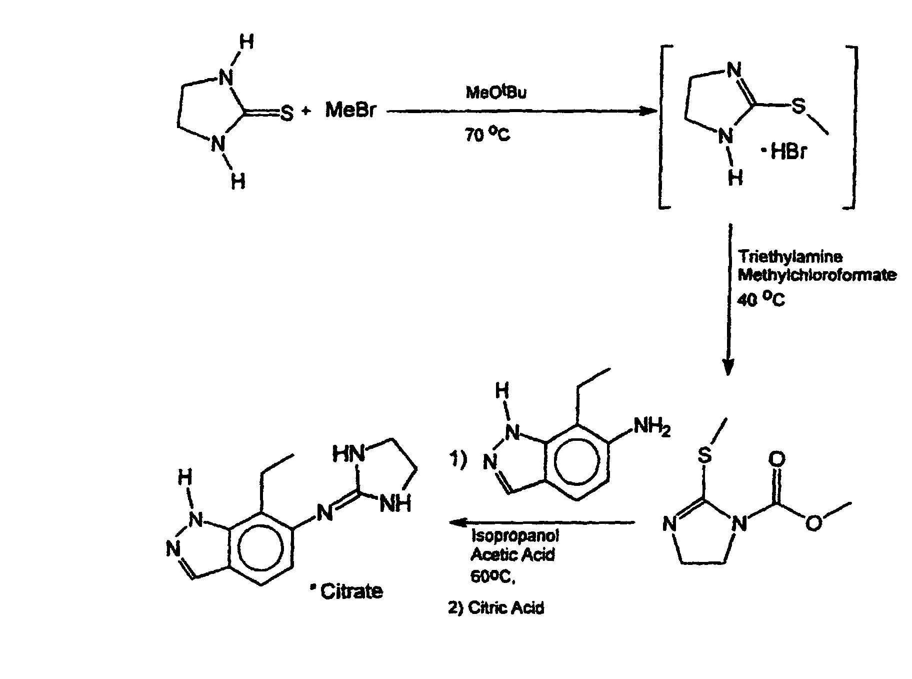

a. 2-Thiomethyl-2-imidazoline hydrobromide:
   In a pressure reactor, methyl bromide (20.5 g) is slowly added to a stirred solution of 2-imidazolidinethione (15 g) in methyl *tert*-butyl ether (120 mL) at ambient temperature. The reaction mixture is heated to 70 °C, under pressure, until the formation of 2-thiomethyl-2-imidazoline hydrobromide is complete.
b. N-Carbomethoxy-2-thiomethyl-2-imidazoline:
   The reaction mixture of step (a) is allowed to cool to ambient temperature and the excess methyl bromide is released and trapped. To this mixture is added triethylamine (53.2 mL), followed by the addition of methylchloroformate (13.6 mL). The reaction mixture is stirred at 40 °C until complete, whereupon the reaction mixture is heated to 55 °C and the hot solution is filtered to remove the insoluble salts. These salts are washed with cold methyl *tert*-butyl ether. The filtrate (and wash solution) is cooled to -20 °C and stirred for 2 hours. The solid obtained is filtered, and washed with cold water and then cold absolute ethanol. The product is dried overnight under vacuum at room temperature to provide N-carbomethoxy-2-thiomethyl-2-imidazoline as a solid.
c. 7-Ethyl-6-(2-imidazolinylamino)indazole, citrate salt:
   6-Amino-7-ethylindazole (9.9 g) (as prepared in Example I of copending U.S. Patent application serial No. 60/031,740) and N-carbomethoxy-2-thiomethyl-2-imidazoline (14 g) are dissolved in 10% acetic acid in isopropanol (210 mL, w/w) and stirred at 60 °C until the reaction is complete. The reaction mixture is cooled to ambient temperature and citric acid (41.5 g) is added. The resulting mixture is stirred for 2 hours. The solution is cooled to -20 °C and stirred until the product has completely precipitated. The crude product so obtained is filtered, recrystallized from ethanol/water, and dried (vacuum, 40 °C) to provide the desired, purified 7-ethyl-6-(2-imidazolinylamino)indazole, citrate salt.

### Example 12

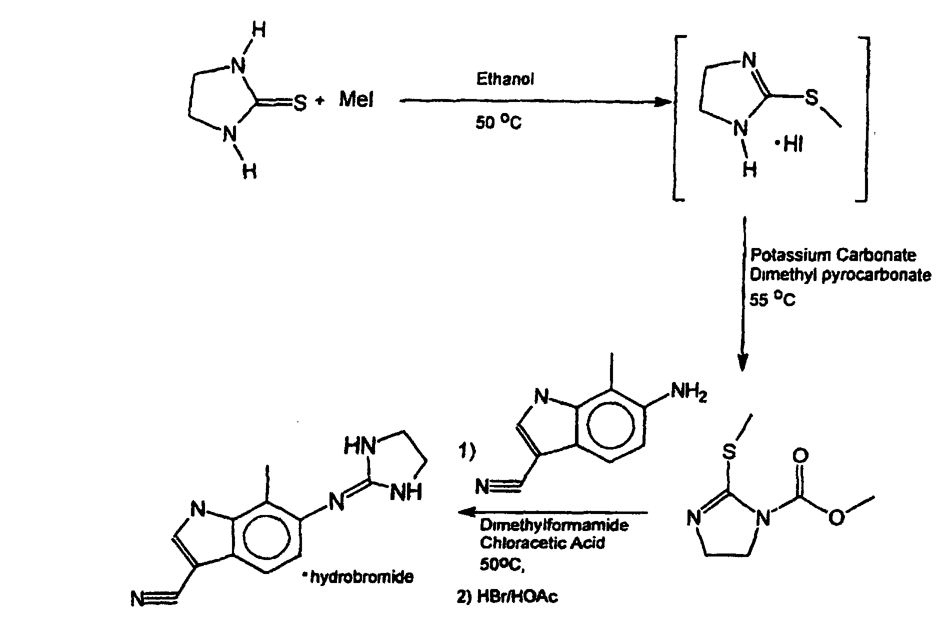

a. 2-Thiomethyl-2-imidazoline hydroiodide:
   Methyl iodide (91 mL) is slowly added to a solution of 2-imidazolidinethione (210 g) in ethanol (1.2 L) with stirring at ambient temperature. The reaction mixture is heated to 40 °C until the formation of 2-thiomethyl-2-imidazoline hydroiodide is complete.
b. N-Carbomethoxy-2-thiomethyl-2-imidazoline:
   The reaction mixture of step (a) is allowed to cool to ambient temperature, whereupon potassium carbonate (426 g) is added, followed by the addition of dimethyl pyrocarbonate (442 mL). The reaction mixture is heated to 55 °C and stirred until complete. The hot solution is filtered to remove the insoluble salts. These salts are washed with cold absolute ethanol. The filtrate (and ethanol wash) is cooled to -20 °C and stirred for 2 hours. The solid obtained is filtered, and washed with cold water, followed by cold absolute ethanol. The product is dried under vacuum at room temperature to provide N-carbomethoxy-2-thiomethyl-2-imidazoline as a solid.
c. 3-Cyano-6-(2-imidazolinylamino)-7-methylindole, hydrobromide salt:
   6-Amino-3-cyano-7-methylindole (34 g) (as prepared in Example 3 of copending U.S. Patent application serial No. 60/031,774) and N-carbomethoxy-2-thiomethyl-2-imidazoline (38 g), are dissolved in 10% chloroacetic acid in N,N-dimethylformamide (480 mL, w/w) and stirred at 50 °C until the reaction is complete. This solution is cooled to ambient temperature and 30% HBr in acetic acid (140 mL) is added, and the mixture is stirred for an additional 4 hours. The resulting solution is cooled to -20 °C and stirred until the product precipitates. The crude product is filtered and recrystallized from ethanol/water to provide the desired, purified 3-cyano-6-(2-imidazolinylamino)-7-methylindole, hydrobromide salt.

### Example 13

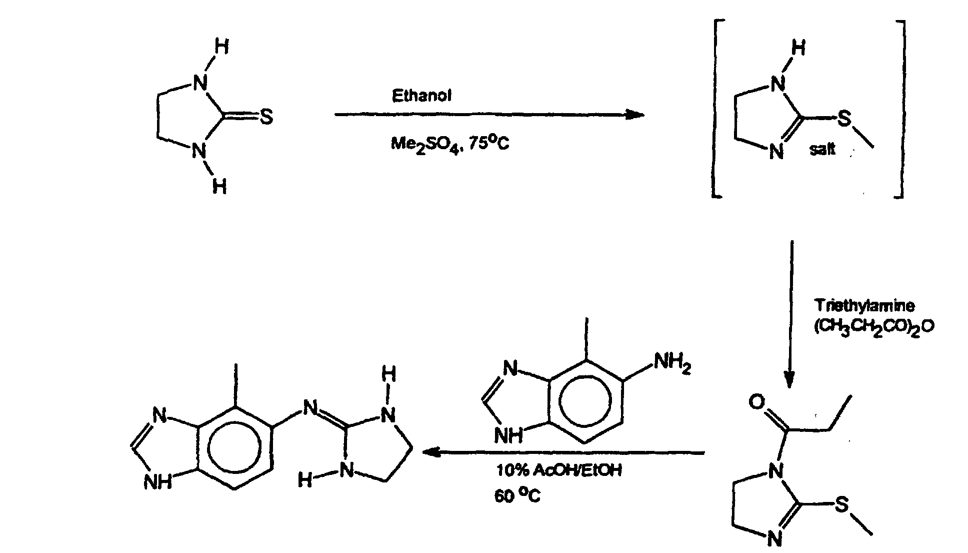

a. 2-Thiomethyl-2-imidazoline:
   Dimethylsulfate (200 mL) is added to a stirred solution of 2-imidazolidinethione (200 g) in ethanol (2 L). The reaction mixture is heated to 75 °C, until the formation of 2-thiomethyl-2-imidazoline is complete.
b. N-Propionyl-2-thiomethyl-2-imidazoline:
   The mixture in step (a) is cooled to ambient temperature, and to this stirred solution is added triethylamine (1.36 L), followed by propionic anhydride (360 mL). The reaction mixture is stirred until complete, whereupon the mixture is heated to 50 °C. The hot solution is filtered to remove the insoluble salts and the salts are washed with cold absolute ethanol. The combined filtrates are cooled to -20 °C and stirred for 2 hours. The solid obtained is filtered, and washed with cold water and then cold absolute ethanol. The product is dried under vacuum at room temperature to provide N-propionyl-2-thiomethyl-2-imidazoline as a solid.
c. 4-Methyl-5-(2-imidazolinylamino)benzimidazole, acetate salt.
   N-Propionyl-2-thiomethyl-2-imidazoline (206.4 g) is combined with 5-amino-4-methylbenzimidazole (147 g) (prepared by deprotecting *tert*-butoxycarbonyl protecting group of intermediate prepared in U.S. Patent No. 5,478,858 issued to Cupps and Bogdan, Dec. 26, 1995 (incorporated herein by reference), under standard deprotection conditions known in the art) in a 10% solution of acetic acid in ethanol (3 L). The mixture is heated to 60 °C until the reaction is complete. The mixture is then cooled to -20 °C and strirred until the product has completely precipitated. The crude product so obtained is filtered, recrystallized from ethanol/water, and dried (vacuum, ambient temperature) to provide the desired, purified 4-methyl-5-(2-imidazolinylamino)-benzimidazole, acetate salt.

### Example 14

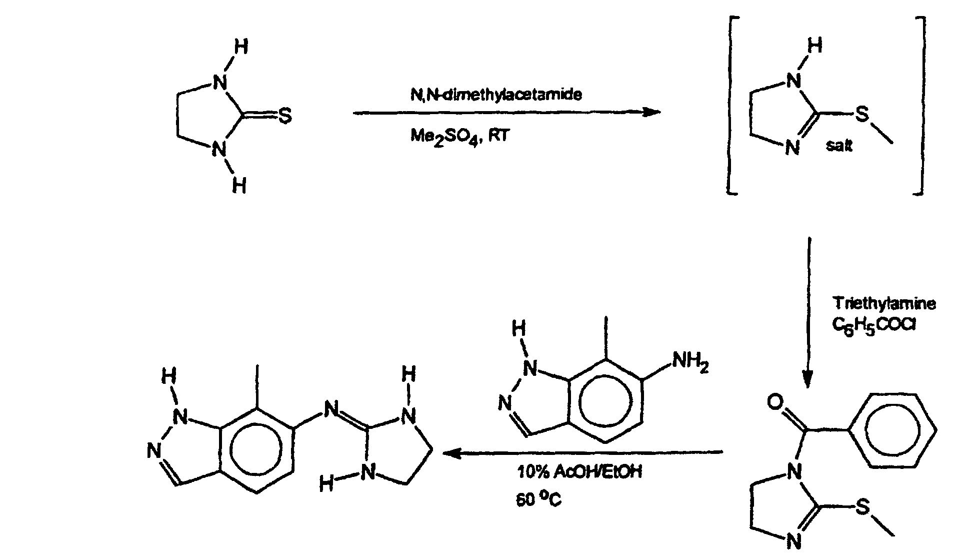

a. 2-Thiomethyl-2-imidazoline:
   Dimethylsulfate (693 g) is added to a stirred mixture of 2-imidazolidinethione (500 g) in dimethylacetamide (5 L). The reaction mixture is stirred at ambient temperature until the formation of 2-thiomethyl-2-imidazoline is complete.
b. N-Benzoyl-2-thiomethyl-2-imidazoline:
   The mixture in step (a) is cooled ambient temperature and to this stirred solution is added triethylamine (2.47 kg) followed by benzoyl chloride (964 g). The reaction mixture at ambient temperature until complete. The reaction mixture is added to cold water and the precipitate that forms is filtered and rinsed twice with cold water. The solid obtained is dried (vacuum, ambient temperature) to provide N-benzoyl-2-thiomethyl-2-imidazoline.
c. 7-Methyl-6-(2-imidazolinylamino)indazole hydrochloride:
   N-Benzoyl-2-thiomethyl-2-imidazoline (264 g) is combined with 6-amino-7-methylindazole (147 g) (as prepared in Example 2 of copending U.S. Patent app. ser. No. 60/031,740) in a 10% solution of acetic acid in ethanol (3 L). The mixture is heated to 60 °C until complete. The reaction mixture is cooled to room temperature and hydrogen chloride gas (128 g) is added. The mixture is stirred at ambient temperature for 2 hours, then cooled to -20 °C and stirred until precipitation of the product is complete. The crude product is recrystallized from methyl *tert*-butyl ether/methanol to provide the desired, purified 7-methyl-6-(2-imidazolinylamino) indazole, hydrochloride salt.

### Example 15

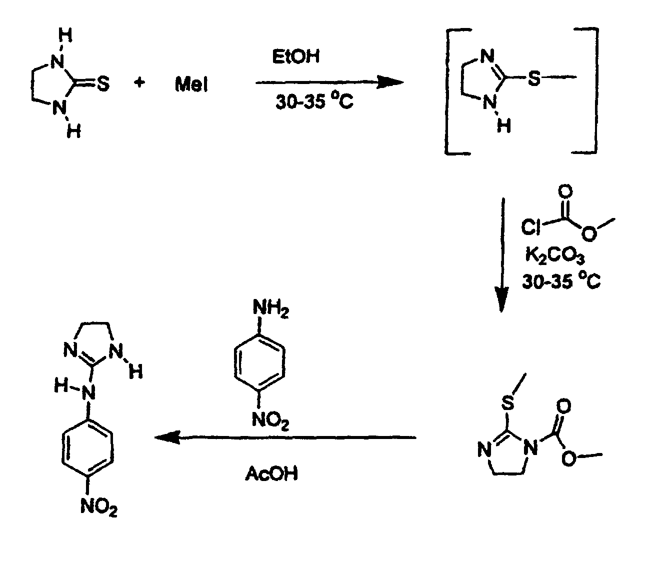

### [(4-Nitrophenyl)amino]-2-imidazoline acetate salt:

4-Nitroaniline (2 g) and N-methoxycarbonyl-2-thiomethyl-2-imidazoline (3.15 g)(prepared as described in Example 2) are dissolved in glacial acetic acid (40 mL), and the reaction mixture is stirred at 60 to 70°C until coupling step is complete. The reaction mixture is diluted with methanol (20 mL), refluxed until deprotection is complete, and then concentrated under reduced pressure. The resulting residue is crystallized from ethyl acetate and hexane to furnish [(4-nitrophenyl)amino]-2-imidazoline, mono acetate salt.

### Example 16

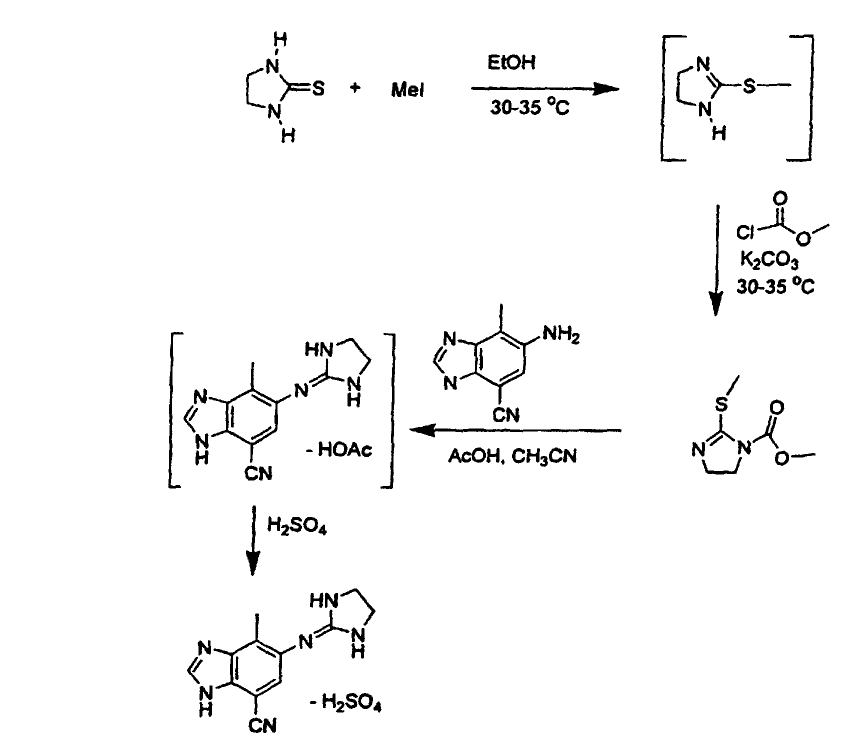

### N-(4,5-Dihydro-1H-imidazol-2-yl)-7-cyano-4-methyl-1H-benzimidazol-5-amine, Sulfuric Acid Salt:

5-Amino-7-cyano-4-methylbenzimidazole (4 g) is prepared treating a heterogeneous solution of 7-cyano-4-methyl-5-nitrobenzimidazole (0.91 g, 0.0045 mol) and 10% Pd/C (100 mg) in methanol (200 mL) with an atmosphere of H₂ (1 atm, balloon) for 14 hr. The resulting mixture is filtered through celite and concentrated *via* rotary evaporation to give rise to a yellow residue. This residue is chromatographed (silica gel, 95:5 ethyl acetate:methanol) to give rise to 5-amino-7-cyano-4-methylbenzimidazole. 5-amino-7-cyano-4-methylbenzinidazole N-methoxycarbonyl-2-thiomethyl-2-imidazoline (4.45 g, 1.1 eq.) (prepared as described in Example 2) are dissolved in acetonitrile (100 mL) and glacial acetic acid (10 mL), and the reaction mixture is stirred at 70°C until the coupling step is complete. The reaction mixture is diluted with methanol (50 mL) and refluxed until deprotection is complete, and then the acetonitrile is evaporated under reduced pressure. The acetic acid solution obtained is dissolved in water (9.25 mL) and the resulting mixture is cooled to 0°C. A 5 molar aqueous solution of H₂SO₄ (5.1 mL) is added dropwise to the cold mixture. The solution is then heated to 65°C and absolute ethanol is added until cloudiness is observed. The mixture is allowed to come to room temperature and is then cooled to 5°C. The solid obtained is filtered, washed with ethanol and dried to provide N-(4,5-dihydro-1H-imidazol-2-yl)-7-cyano-4-methyl-1H-benzimidazol-5-amine as its sulfuric acid salt.

### Example 17

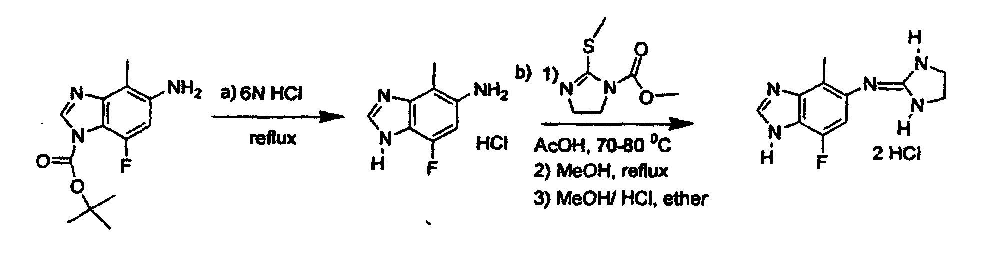

a. 4-Methyl-5-amino-7-fluorobenzunidazole hydrochloride:
   1-tert-Butoxycarbonyl-4-methyl-5-amino-7-fluorobenzimidazole (1g) (prepared as described in U.S. Patent No. 5,478,858 issued to Cupps and Bogdan, Dec. 26, 1995, incorporated herein by reference) and 6N HCl (10 mL) are combined and heated to reflux while stirring. After completion of the t-BOC group deprotection, the reaction mixture is concentrated under reduced pressure and dried to furnish 4-methyl-5-amino-7-fluorobenzimidazole hydrochloride.
b. 4-Methyl-7-fluoro-5-(-2-imidazolinylamino) benzimidazole dihydrochloride:
   To the solid obtained in step a is added N-methoxycarbonyl-2-thiomethyl-2-imidazoline (0.78 g) and glacial acetic acid (20 mL). The mixture is stirred at 60 to 70°C until the coupling step is complete. The reaction mixture is then diluted with methanol (10 mL), refluxed until deprotection is complete, and then concentrated under reduced pressure. The resulting residue is diluted with methanolic HCl (20 mL) and then treated with anhydrous ether to precipitate 4-methyl-7-fluoro-5-(2-imidazolinylamino)benzimidazole dihydrochloride.

## Claims

1. A method of making a 2-amino-2-imidazoline, guanidine, or 2-amino-3,4,5,6-tetrahydropyrimidine derivative having a general structure: 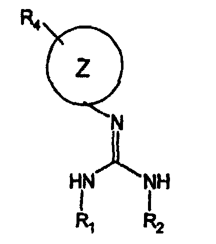 or the tautomers thereof, wherein:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring;
(c) Z is a saturated, unsaturated or aromatic, monocyclic or polycyclic carbocycle or heterocycle containing one or more heteroatoms selected from O, N, or S; and
(d) R₄ is one or more substituents on the Z ring comprising independently hydrogen, alkoxy, alkylthio, alkyl, alkenyl, amino, cyano, halogen, hydroxy, nitro, and thiol;
(e) or a salt or pharmaceutically-acceptable salt, thereof;
which comprises the steps of:
(I) preparing an intermediate having the general structure: 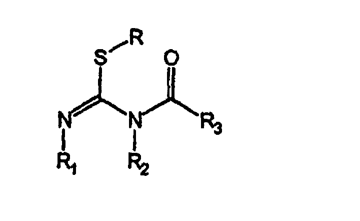 wherein:
(a) R is selected from the group consisting of methyl, ethyl, and benzyl;
(b) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(c) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring;
(d) R₃ is -O-R₅ or -R₆;
(e) R₅ is selected from the group consisting of methyl, ethyl, benzyl, and *tert*-butyl; and
(f) R₆ is selected from the group consisting of methyl, ethyl, *tert*-butyl, and phenyl;
from a thiourea having the general structure: 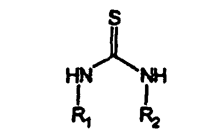 wherein:
(a) R₁ is methyl, ethyl, a methylene group connected to R₂ through a single bond such that R₁ and R₂ form a five-membered ring, or a methylene group connected to R₂ through another methylene group such that R₁ and R₂ form a six-membered ring;
(b) R₂ is methyl, ethyl, a methylene group connected to R₁ through a single bond such that R₁ and R₂ form a five-membered ring, or a methylene group connected to R₁ through another methylene group such that R₁ and R₂ form a six-membered ring;
in a two-step, one-pot reaction by:
a) alkylating the thiourea using an alkylating agent to form a 2-thio-substituted-2-imidazoline, 2-thioalkyl-2-guanidine, or 2-thioalkyl-(3,4,5,6-tetrahydro)-pyrimidine;
b) acylating the 2-thio-substituted-2-imidazoline, 2-thioalkyl-2-guanidine, or 2-thioalkyl-(3,4,5,6-tetrahydro)-pyrimidine of step (I)(a) with an acylating agent in the presence of a base; and
(II) coupling the intermediate of step (I) with an amine of structure: 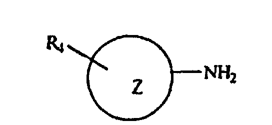 in the presence of an organic acid.

2. A method of making a 2-amino-2-imidazoline, guanidine, or 2-amino-3,4,5,6-tetrahydropyrimidine derivative according to Claim 1 wherein the organic acid of step (II) is selected from the group consisting of formic acid, acetic acid, chloroacetic acid, dichloroacetic acid, propionic acid, benzoic acid, maleic acid, fumaric acid, succinic acid, and tartaric acid, preferably acetic acid, propionic acid and chloroacetic acid.

3. A method of making a 2-amino-2-imidazoline, guanidine, or 2-amino-3,4,5,6-tetrahydropyrimidine derivative according to Claim 1 or 2 wherein the alkylating agent of step (I)(a) is selected from the group consisting of methyl iodide, methyl bromide, methyl chloride, dimethyl sulfate, ethyl iodide, ethyl bromide, ethyl chloride, diethyl sulfate, and benzyl bromide, preferably methyl iodide, methybromide, dimethyl, sulfate, ethyl iodide, and diethyl sulfate.

4. A method of making a 2-amino-2-imidazoline, guanidine, or 2-amino-3,4,5,6-tetrahydropyrimidine derivative according to any one of the preceding claims wherein the base of step (I)(b) is selected from the group consisting of triethylamine, trimethylamine, 4-dimethylaminopyridine, pyridine, potassium carbonate, sodium carbonate, potassium bicarbonate, and sodium bicarbonate, preferably trimethylamine, triethylamine and potassium carbonate.

5. A method of making a 2-amino-2-imidazoline, guanidine, or 2-amino-3,4,5,6-tetrahydropyrimidine derivative according to any one of the preceding claims wherein the acylating agent of step (I)(b) is selected from the group consisting of di-*tert*-butyl dicarbonate, diethylpyrocarbonate, dimethylpyrocarbonate, methyl chloroformate, ethyl chloroformate, acetyl chloride, propionyl chloride, acetic anhydride, propionic anhydride, trimethylacetyl chloride, trimethylacetic anhydride, and benzoyl chloride, preferably di-tert-butyl dicarbonate, dimethylpyrocarbonate, and methyl chloroformate.

6. A method of making a 2-amino-2-imidazoline, guanidine, or 2-amino-3,4,5,6-tetrahydropyrimidine derivative according to any one of the preceding claims wherein the alkylation of step I(a) and the acylation of step I(b) are carried out in the presence of an organic solvent selected from the group consisting of methyl *tert*-butyl ether, ethyl acetate, methanol, ethanol, 2-propanol, and N,N-dimethylacetamide.

7. A method of making a 2-amino-2-imidazoline, guanidine, or 2-amino-3,4,5,6-tetrahydropyrimidine derivative according to any one of the preceding claims wherein the coupling of step (II) is carried out at a temperature from ambient temperature to 150°C in the presence of an organic solvent selected from the group consisting of methanol, ethanol, 2-propanol, butanol, *sec*-butanol, and isoamyl alcohol, preferably acetic acid.

8. A method of making a 2-amino-2-imidazoline, guanidine, or 2-amino-3,4,5,6-tetrahydropyrimidine derivative according to any one of the preceding claims wherein the alkylating agent of step (I)(a) is selected from the group consisting of methyl iodide and dimethyl sulfate, and the acylating agent of Step (I)(b) is methyl chloroformate.

9. A method according to any one of the preceding claims wherein the derivative is a 2-amino-2-imidazoline.

10. A method of making a 2-amino-2-imidazoline according to any one of the preceding claims wherein the amine of step (II) is 5-amino-4-ethylbenzimidazole.

## Patentansprüche

1. Verfahren zur Herstellung eines 2-Amino-2-imidazolin-, Guanidin- oder 2-Amino-3,4,5,6-tetrahydropryrimidin-Derivats mit einer allgemeinen Struktur: 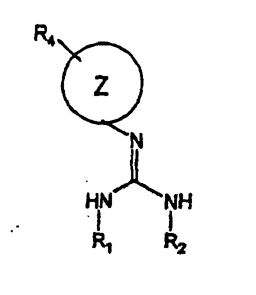 oder die Tautomeren hiervon, worin:
(a) R₁ Methyl, Ethyl, eine Methylengruppe, verbunden mit R₂ über eine Einfachbindung, sodass R₁ und R₂ einen fünfgliedrigen Ring bilden, oder eine Methylengruppe. verbunden mit R₂ über eine andere Methylengruppe, sodass R₁ und R₂einen sechsgliedrigen Ring bilden, ist;
(b) R₂ Methyl, Ethyl, eine Methylengruppe, verbunden mit R₁ über eine Einfachbindung, sodass R₁ und R₂ einen fünfgliedrigen Ring bilden, oder eine Methylengruppe, verbunden mit R₁ über eine andere Methylengruppe, sodass R₁ und R₂ einen sechsgliedrigen Ring bilden, ist;
(c) Z ein gesättigter, ungesättigter oder aromatischer, monozyklischer oder polyzyklischer Carbozyklus oder Heterozyklus ist, enthaltend ein oder mehrere aus O, N oder S gewählte Heteroatome; und
(d) R₄ ein oder mehrere Substituenten an dem Z-Ring ist, umfassend unabhängig Wasserstoff, Alkoxy, Alkylthio, Alkyl, Alkenyl, Amino, Cyano, Halogen, Hydroxy, Nitro und Thiol;
(e) oder ein Salz oder pharmazeutisch annehmbares Salz hiervon;
welches Verfahren die Schritte umfasst:
(I) Herstellen einer Zwischenverbindung mit der allgemeinen Struktur: 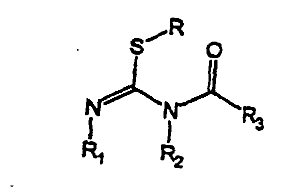 worin:
(a) R aus der Gruppe gewählt ist, bestehend aus Methyl, Ethyl und Benzyl;
(b) R₁ Methyl, Ethyl, eine Methylengruppe, verbunden mit R₂ über eine Einfachbindung, sodass R₁ und R₂ einen fünfgliedrigen Ring bilden, oder eine Methylengruppe, verbunden mit R₂ über eine andere Methylengruppe, sodass R₁ und R₂ einen sechsgliedrigen Ring bilden, ist;
(c) R₂ Methyl, Ethyl, eine Methylengruppe, verbunden mit R₁ über eine Einfachbindung, sodass R₁ und R₂ einen fünfgliedrigen Ring bilden, oder eine Methylengruppe, verbunden mit R₁ über eine andere Methylengruppe, sodass R₁ und R₂ einen sechsgliedrigen Ring bilden, ist;
(d) R₃ -O-R₅ oder -R₆ ist;
(e) R₅ aus der Methyl, Ethyl. Benzyl und tert-Butyl bestehenden. Gruppe gewählt ist; und
(f) R₆ aus der Methyl. Ethyl, tert-Butyl und Phenyl bestehenden Gruppe gewählt ist;
aus einem Thioharnstoff mit der allgemeinen Struktur: 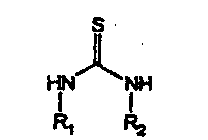 worin:
(a) R₁ Methyl, Ethyl, eine Methylengruppe, verbunden mit R₂ über eine Einfachbindung, sodass R₁ und R₂ einen fünfgliedrigen Ring bilden, oder eine Methylengruppe, verbunden mit R₂ über eine andere Metbylengruppe. sodass R₁ und R₂ einen sechsgliedrigen Ring bilden, ist;
(b) R₂ Methyl, Ethyl, eine Methylengruppe, verbunden mit R₁ über eine Einfachbindung, sodass R₁ und R₂ einen fünfglledrigen Ring bilden, oder eine Methylengruppe, verbunden mit R₁ über eine andere Methylengruppe, sodass R₁ und R₂ einen sechsgliedrigen Ring bilden, ist;
in einer Zweistufen-Eintopfreaktion durch:
a) Alkylieren des Thioharnstoffs unter Verwendung eines Alkylierungsmittels zur Bildung eines 2-thiosubstituierten-2-Imidazolins, 2-Thioalkyl-2-guanidins oder 2-Thioalkyl-3,4,5,6-tetrahydro-pyrimidins;
b) Acylieren des 2-thiosubstituierten-2-Imidazolins, 2-Thioalkyl-2-guanidins oder 2-Thioalkyl-3,4,5,6-tetrahydro-pyrimidins aus Schritt (I)(a) mit einem Acylierungsmittel in Gegenwart einer Base; und
(II) Kuppeln der Zwischenverbindung aus Schritt (I) mit einem Amin der Struktur: 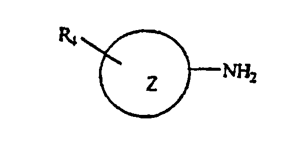 in Gegenwart einer organischen Säure.

2. Verfahren zur Herstellung eines 2-Amino-2-imidazolin-, Guanidin- oder 2-Amino-3,4,5,6-tetrahydropryrimidin-Derivats nach Anspruch 1, wobei die organische Säure aus Schritt (II) aus der Gruppe gewählt ist, bestehend aus Ameisensäure, Essigsäure, Chloressigsäure, Dichloressigsäure, Propionsäure, Benzoesäure, Maleinsäure, Fumarsäure, Bernsteinsäure und Weinsäure, vorzugsweise Essigsäure, Propionsäure und Chloressigsäure.

3. Verfahren zur Herstellung eines 2-Amino-2-imidazolin-, Guanidin- oder 2-Amino-3,4,5,6-tetrahydropryrimidin-Derivats nach Anspruch 1 oder 2, wobei das Alkylierungsmittel aus Schritt (I)(a) aus der Gruppe gewählt ist, bestehend aus Methyllodid, Methylbromid, Methylchlorid, Dimethylsulfat, Ethyllodid, Ethylbromid, Ethylchlorid, Diethylsulfat und Benzylbromid, vorzugsweise Methyljodid, Methylbromid, Dimethylsulfat, Ethyljodid und Diethylsulfat.

4. Verfahren zur Herstellung eines 2-Amino-2-imidazolin-, Guanidin- oder 2-Amino-3,4,5,6-tetrahydropryrimidin-Derivats nach mindestens einem der vorangehenden Ansprüche, wobei die Base aus Schritt (I)(b) aus der Gruppe gewählt ist, bestehend aus Triethylamin, Trimethylamin, 4-Dimethylaminopyridin, Pyridin. Kaliumcarbonat, Natriumcarbonat, Kaliumbicarbonat und Natriumbicarbonat, vorzugsweise Trimethylamin, Triethylamin und Kaliumcarbonat.

5. Verfahren zur Herstellung eines 2-Amino-2-imidazolin-, Guanidin- oder 2-Amino-3,4,5,6-tetrahydropryrimidin-Derivats nach mindestens einem der vorangehenden Ansprüche, wobei das Acylierungsmittel aus Schritt (I)(b) aus der Gruppe gewählt ist, bestehend aus Di-tert-butyldicarbonat, Diethylpyrocarbonat, Dimethylpyrocarbonat. Methylchlorformiat, Ethylchlorformiat, Acetylchlorid, Propionylchlorid. Essigsäureanhydrid, Propionsäureanhydrid, Trimethylacetylchlorid, Trimethylessigsäureanhydrid und Benzoylchlorid, vorzugsweise Di-tert-butyldicarbonat, Dimethylpyrocarbonat und Methylchlorformiat.

6. Verfahren zur Herstellung eines 2-Amino-2-imidazolin-, Guanidin- oder 2-Amino-3,4,5,6-tetrahydropryrimidin-Derivats nach mindestens einem der vorangehenden Ansprüche, wobei die Alkylierung in Schritt I(a) und die Acylierung in Schritt I(b) in Gegenwart eines organischen Lösungsmittels durchgeführt werden, gewählt aus der Methyl-tert-butylether, Ethylacetat, Methanol, Ethanol, 2-Propanol und N,N-Dirnethylacetamid bestehenden Gruppe.

7. Verfahren zur Herstellung eines 2-Amino-2-imidazolin-, Guanidin- oder 2-Amino-3,4,5,6-tetrahydropryrimidin-Derivats nach mindestens einem der vorangehenden Ansprüche, wobei die Kupplung in Schritt (II) bei einer Temperatur von Umgebungstemperatur bis 150°C in Gegenwart eines organischen Lösungsmittels durchgeführt wird, gewählt aus der Methanol, Ethanol, 2-Propanol, Butanol, sec-Butanol und Isoamylalkohol, vorzugsweise Essigsäure, bestehenden. Gruppe.

8. Verfahren zur Herstellung eines 2-Amino-2-imidazolin-, Guanidin- oder 2-Amino-3,4,5,6-tetrahydropryrimidin-Derivats nach mindestens einem der vorangehenden Ansprüche, wobei das Alkylierungsmittel in Schritt (I)(a) aus der Gruppe gewählt ist, bestehend aus Methyljodid und Dimethylsulfat, und das Acylierungsmittel aus Schritt (I)(b) Methylchlorformiat ist.

9. Verfahren nach mindestens einem der vorangehenden Ansprüche, wobei das Derivat ein 2-Amino-2-imidazolin ist.

10. Verfahren zur Herstellung eines 2-Amino-2-imidazolins nach mindestens einem der vorangehenden Ansprüche, wobei das Amin aus Schritt (II) 5-Amlno-4-ethylbenzimidazol ist.

## Revendications

1. Procédé de fabrication d'un dérivé 2-amino-2-imidazoline, guanidine ou 2-amino-3,4,5,6-tétrahydropyrimidine ayant pour structure générale : 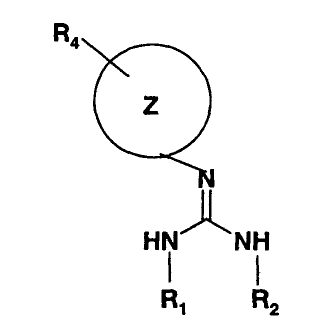 ou les tautomères de celui-ci, dans laquelle :
(a) R₁ est un groupe méthyle, éthyle, un groupe méthylène lié à R₂ par une simple liaison, de sorte que R₁ et R₂ forment un cycle à cinq membres, ou un groupe méthylène lié à R₁ par un autre groupe méthylène de sorte que R₁ et R₂ forment un cycle à six membres;
(b) R₂ est un groupe méthyle, éthyle, un groupe méthylène lié à R₁ par une simple liaison de sorte que R₁ et R₂ forment un cycle à cinq membres, ou un groupe méthylène lié à R₂ par un autre groupe méthylène de sorte que R₁ et R₂ forment un cycle à six membres;
(c) Z est un carbocycle ou hétérocycle monocyclique ou polycyclique saturé, insaturé ou aromatique, contenant un ou plusieurs hétéroatomes choisis parmi O, N, ou S; et
(d) R₄ représente un ou plusieurs substituants sur le cycle Z comprenant indépendamment un atome d'hydrogène, un groupe alcoxy, alkylthio, alkyle, alcényle, amino, cyano, halogéno, hydroxy, nitro, et thiol;
(e) ou un sel, ou un sel acceptable sur le plan pharmaceutique de celui-ci;
qui comprend les étapes de :
(I) préparation d'un intermédiaire ayant pour structure générale : 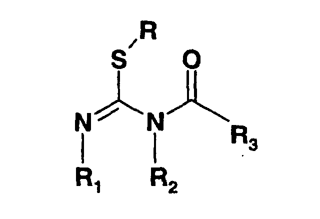 dans laquelle :
(a) R est choisi dans le groupe constitué par un groupe méthyle, éthyle, et benzyle;
(b) R₁ est un groupe méthyle, éthyle, un groupe méthylène lié à R₂ par une simple liaison, de sorte que R₁ et R₂ forment un cycle à cinq membres, ou un groupe méthylène lié à R₂ par un autre groupe méthylène de sorte que R₁ et R₂ forment un cycle à six membres;
(c) R₂ est un groupe méthyle, éthyle, un groupe méthylène lié à R₁ par une simple liaison de sorte que R₁ et R₂ forment un cycle à cinq membres, ou un groupe méthylène lié à R₁ par un autre groupe méthylène de sorte que R₁ et R₂ forment un cycle à six membres;
(d) R₃ est -O-R₅ ou -R₆;
(e) R₅ est choisi dans le groupe constitué par un groupe méthyle, éthyle, benzyle, et *tert-butyle*; et
(f) R₆ est choisi dans le groupe constitué par un groupe méthyle, éthyle, *tert-butyle*, et phényle;
à partir d'une thiourée ayant pour structure générale : 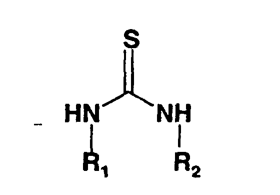
dans laquelle :
(a) R₁ est un groupe méthyle, éthyle, un groupe méthylène lié à R₂ par une simple liaison, de sorte que R₁ et R₂ forment un cycle à cinq membres, ou un groupe méthylène lié à R₂ par un autre groupe méthylène de sorte que R₁ et R₂ forment un cycle à six membres;
(b) R₂ est un groupe méthyle, éthyle, un groupe méthylène lié à R₁ par une simple liaison de sorte que R₁ et R₂ forment un cycle à cinq membres, ou un groupe méthylène lié à R₁ par un autre groupe méthylène de sorte que R₁ et R₂ forment un cycle à six membres; dans une réaction en deux étapes dans un seul récipient au moyen :
(a) d'une alkylation de la thiourée en employant un agent d'alkylation afin former une 2-imidazoline à 2-thio-substitution, une 2-thioalkyl-2-guanidine, ou 2-thioallkyl-3,4,5,6-tétrahydropyrimidine;
(b) d'une acylation de la 2-imidazoline à 2-thio-substitution, de la 2-thioalkyl-2-guanidine, ou de la 2-thioalkyl-3,4,5,6-tétrahydropyrimidine de l'étap (I) (a) avec un agent d'acylation en présence d'une base; et
(II) un couplage de l'intermédiaire de l'étape (I) à une amine de structure : 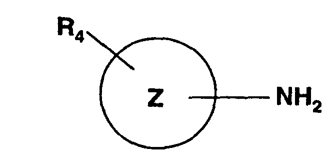 en présence d'un acide organique.

2. Procédé de fabrication d'un dérivé 2-amino-2-imidazoline, guanidine, ou 2-amino-3,4,5,6-tétrahydropyrimidine selon la revendication 1, dans lequel l'acide organique de l'étape (II) est choisi dans le groupe constitué par l'acide formique, l'acide acétique, l'acide chloroacétique, l'acide dichloroacétique, l'acide propionique, l'acide benzoïque, l'acide maléique, l'acide furmarique, l'acide succinique, et l'acide tartrique, de préférence l'acide acétique, l'acide propionique et l'acide chloroacétique.

3. Procédé de fabrication d'un dérivé 2-amino-2-imidazoline, guanidine, ou 2-amino-3,4,5,6-tétrahydropyrimidine selon la revendication 1 ou 2, dans lequel l'agent d'alkylation de l'étape (I)(a) est choisi dans le groupe constitué par l'iodure de méthyle, le bromure de méthyle, le chlorure de méthyle, le sulfate de diméthyle, l'iodure d'éthyle, le bromure d'éthyle, le chlorure d'éthyle, le sulfate de diméthyle, et le bromure de benzyle, de préférence l'iodure de méthyle, le bromure de méthyle, le sulfate de diméthyle, l'iodure d'éthyle et le sulfate de diméthyle.

4. Procédé de fabrication d'un dérivé 2-amino-2-imidazoline, guanidine, ou 2-amino-3,4,5,6-tétrahydropyrimidine selon l'une quelconque des revendications précédentes, dans lequel la base de l'étape (I)(b) est choisie dans le groupe constitué par la triéthylamine, la triméthylamine, la 4-diméthylaminopyridine, la pyridine, le carbonate de potassium, le carbonate de sodium, le bicarbonate de potassium, et le bicarbonate de sodium, de préférence la triméthylamine, la triéthylamine et le carbonate de potassium.

5. Procédé de fabrication d'un dérivé 2-amino-2-imidazoline, guanidine, ou 2-amino-3,4,5,6-tétrahydropyrimidine selon l'une quelconque des revendications précédentes, dans lequel l'agent d'acylation de l'étape (I) (b) est choisi dans le groupe constitué par le dicarbonate de di-*tert*-butyle, le pyrocarbonate de diéthyle, le pyrocarbonate de diméthyle, le chloroformiate de méthyle, le chloroformiate d'éthyle, le chlorure d'acétyle, le chlorure de propionyle, l'anhydride acétique, l'anhydride propionique, le chlorure de triméthylacétyle, l'anhydride triméthylacétique, et le chlorure de benzoyle, de préférence le dicarbonate de di-tert-butyle, le pyrocarbonate de diméthyle, et le chloroformiate de méthyle.

6. Procédé de fabrication d'un dérivé 2-amino-2-imidazoline, guanidine, ou 2-amino-3,4,5,6-tétrahydropyrimidine selon l'une quelconque des revendications précédentes, dans lequel l'alkylation de l'étape (I)(a) et l'acylation de l'étape(I) (b) sont réalisées en présence d'un solvant organique choisi dans le groupe constitué par le tert-butyléther de méthyle, l'acétate d'éthyle, le méthanol, l'éthanol, le 2-propanol, et le N,N-diméthylacétamide.

7. Procédé de fabrication d'un dérivé 2-amino-2-imidazoline, guanidine, ou 2-amino-3,4,5,6-tétrahydropyrimidine selon l'une quelconque des revendications précédentes, dans lequel le couplage de l'étape (II) est réalisé à une température allant de la température ambiante jusqu'à 150°C en présence d'un solvant organique choisi dans le groupe constitué par le méthanol, l'éthanol, le 2-propanol, le butanol, le sec-butanol et l'alcool isoamylique, de préférence l'acide acétique.

8. Procédé de fabrication d'un dérivé 2-amino-2-imidazoline, guanidine, ou 2-amino-3,4,5,6-tétrahydropyrimidine selon l'une quelconque des revendications précédentes, dans lequel l'agent d'alkylation de l'étape (I)(a) est choisi dans le groupe constitué par l'iodure de méthyle, et le sulfate de diméthyle, et l'agent d'acylation de l'étape (I)(b) est le chloroformiate de méthyle.

9. Procédé selon l'une quelconque des revendications précédentes, dans lequel le dérivé est une 2-amino-2-imidazoline.

10. Procédé de fabrication d'une 2-amino-2-imidazoline selon l'une quelconque des revendications précédentes, dans lequel l'amine de l'étape (II) est le 5-amino-4-éthylbenzymidazole.
